# EUROPÄISCHE PATENTSCHRIFT

(11) **EP 2 874 600 B1**
(45) Veröffentlichungstag und Bekanntmachung des Hinweises auf die Patenterteilung: **08.06.2016**
(21) Anmeldenummer: 13745409.6
(22) Anmeldetag: 05.08.2013
(51) Int. Cl.: A61K 9/20, A61K 31/045, A61K 31/728, A61K 31/731

(54) **PHARMAZEUTISCHE ZUSAMMENSETZUNG ZUR BEHANDLUNG VON HEISERKEIT ODER HALSSCHMERZEN**
PHARMACEUTICAL COMPOSITION FOR THE TREATMENT OF CROAKINESS AND SORE THROAT
COMPOSITION PHARMACEUTIQUE POUR LE TRAITEMENT D'ENROUEMENT ET DE MAL À LA GORGE

(30) Priorität: 04.10.2012 DE 102012019414; 17.01.2013 DE 102013000700
(43) Veröffentlichungstag der Anmeldung: 27.05.2015
(73) Patentinhaber: Maria Clementine Martin Klosterfrau Vertriebsgesellschaft mbH, 50670 Köln (DE)
(72) Erfinder: VESTWEBER, Anna-Maria, 51399 Burscheid (DE); GALÁN SOÚSA, José, 13465 Berlin (DE); UNKAUF, Markus, 50670 Köln (DE); PLOCH, Michael, 16565 Lehnitz (DE)
(74) Vertreter: Von Rohr Patentanwälte Partnerschaft mbB
(86) Internationale Anmeldenummer: PCT/EP2013/066364
(87) Internationale Veröffentlichungsnummer: WO 2014/053263

(56) Entgegenhaltungen:
- WO-A2-2007/110871
- DE-U1-202004 016 646

## Beschreibung

Die vorliegende Erfindung betrifft das Gebiet der Medizin, insbesondere der Behandlung von Heiserkeit bzw. entzündlichen Erkrankungen des Mund- und Rachenraums (wie z. B. Halsschmerzen).

Insbesondere betrifft die vorliegende Erfindung eine Zusammensetzung, vorzugsweise eine pharmazeutische Zusammensetzung, welche sich für die topische Behandlung von Heiserkeit bzw. entzündlichen Erkrankungen des Mund- und Rachenraums (wie z. B. Halsschmerzen) eignet. Darüber hinaus betrifft die vorliegende Erfindung die erfindungsgemäßen Verwendungen der Zusammensetzung nach der Erfindung.

Unter Heiserkeit bzw. Dysphonie wird üblicherweise eine Beeinträchtigung des stimmlichen Teils der Phonation (Artikulation) verstanden, wobei in diesem Zusammenhang im Allgemeinen eine Funktionsstörung des Kehlkopfes und des Ansatzrohres vorliegt. Die Stimme klingt bei Heiserkeit je nach Befund und Ausprägung heiser, rau, belegt oder behaucht und ist nicht mehr "flexibel", d. h. Klangfarbe, Tonhöhe und Lautstärke können vom Betroffenen nur im verminderten Umfang variiert werden. Insbesondere tritt bei Heiserkeit oftmals auch das Symptom der Mundtrockenheit (Xerostomie) auf

Die der Heiserkeit zugrundeliegenden Ursachen sind vielfältig und können sowohl funktionell als auch organisch bedingt sein. Zu den funktionellen Ursachen gehören insbesondere eine allgemeine Überbeanspruchung der Stimme, beispielsweise durch Singen, oder stimmschädigende Sprechgewohnheiten. Zu den häufigsten organischen Ursachen gehören insbesondere entzündliche Erkrankungen des Mund- und Rachenraums, wie sie beispielsweise bei Erkältungen oder grippalen Infekten auftreten. Darüber hinaus kommen als organische Ursachen für Heiserkeit auch Lähmungen oder Gewebsneubildungen in Betracht. In seltenen Fällen ist es auch möglich, dass angeborene Fehlbildungen im Bereich des Kehlkopfes zu Heiserkeit führen.

Sowohl durch funktionelle als auch durch organische Ursachen, insbesondere entzündliche Erkrankungen des Mund- und Rachenraums, hervorgerufene Heiserkeit ist für den betroffenen Patienten oftmals mit einer unangenehmen Schmerz- und Entzündungssymptomatik verbunden. Auch können schmerzhafte Schluckbeschwerden auftreten, was insbesondere die Nahrungsaufnahme erschwert. Betroffene empfinden darüber hinaus die Beeinträchtigung der Phonation, welche charakteristisch für Heiserkeit ist, als äußerst störend.

Die Behandlung von Heiserkeit, sowohl bei funktionellen als auch bei organischen Ursachen, insbesondere entzündlichen Erkrankungen des Mund-und Rachenraums, erfolgt üblicherweise - in Abhängigkeit von der Schwere des Krankheitsbildes - durch topische Therapie.

Mit den im Stand der Technik bekannten Maßnahmen lässt sich jedoch oftmals kein zufriedenstellender Therapieerfolg erzielen, wie im Folgenden beschrieben.

Die Therapiemaßnahmen beschränken sich nämlich im Allgemeinen auf die Gabe von "Hausmitteln", wie beispielsweise heißer Milch mit Honig oder Gurgellösungen auf Basis von Kochsalz oder Salbei. Derartige Behandlungsmethoden gewährleisten jedoch oftmals keine zufriedenstellende Linderung der Beschwerden. Einige der altbekannten Hausmittel, wie insbesondere heiße Milch mit Honig, sind sogar abträglich, da sie eine verstärkte Schleimbildung im Hals bewirken und somit dem Heilungsprozess bzw. der Regeneration entgegenstehen.

Darüber hinaus kommen häufig handelsübliche Lutschtabletten zum Einsatz, welche zwar eine kurzzeitige Linderung verschaffen, deren Wirkung jedoch üblicherweise äußerst schnell nachlässt.

Insbesondere bieten sämtliche im Stand der Technik angewandten Behandlungskonzepte für Heiserkeit keine ausreichende Linderung der Schmerz- und Entzündungssymptomatik, vor allem wenn der Heiserkeit organische Ursachen, wie entzündliche Erkrankungen des Mund- und Rachenraums, zugrundeliegen.

Entsprechendes gilt auch für die Behandlung von Halsschmerzen oder anderen entzündlichen Erkrankungen des Mund- und Rachenraums. Auch hier sind die Therapeutika des Standes der Technik oftmals nicht effizient.

Entzündliche Erkrankungen des Mund- und Rachenraums, d. h. Entzündungen im Bereich der Mundhöhle und des Hals-/Rachenraums, treten oftmals als Begleiterscheinung von Erkältungserkrankungen und grippalen Infekten, aber auch als eigenständige Erkrankungen auf. Solche entzündlichen Prozesse des Mund- und Rachenraums sind oftmals mit einer für den betroffenen Patienten unangenehmen Schmerzsymptomatik verbunden. Nicht beschränkende Beispiele für derartige entzündliche Erkrankungen des Hals-/Rachenraums sind Halsentzündungen, insbesondere Angina, Entzündungen des Kehlkopfs (Laryngitis), Entzündungen der Rachenschleimhaut (Pharyngitis) und Entzündungen der Rachenmandeln (Tonsillitis). Solche Erkrankungen des Hals-/Rachenraums sind oftmals von schmerzhaften Schluckbeschwerden und Halsschmerzen begleitet. Auch Entzündungen im Bereich der Mundhöhle, wie z. B. Stomatitis, Gingivitis, Mundschleimhautläsionen und dergleichen, sind oftmals von Schmerzen begleitet. Für weitere diesbezügliche Einzelheiten zu den vorgenannten Erkrankungen kann beispielsweise auf Roche-Lexikon Medizin, 3. Auflage, 1993, Urban & Schwarzenberg, München/Wien/Baltimore, sowie auf Pschyrembel, Medizinisches Wörterbuch, 257. Auflage, 1993, Nikol Verlagsgesellschaft mbH, Hamburg, verwiesen werden. Der Begriff "entzündliche Erkrankungen des Mund- und Rachenraums" ist somit sehr weit zu verstehen und umfasst sämtliche entzündlichen Erkrankungen, welche im Bereich der Mundhöhle, des Rachens und des Halsraums auftreten können.

Die Behandlung entzündlicher Erkrankungen des Mund- und Rachenraums erfolgt - in Abhängigkeit von der Schwere des Krankheitsbildes - im allgemeinen durch topische und gegebenenfalls zusätzliche systemische Therapie in schwerwiegenderen Fällen. Während in schwereren Fällen systemisch Antibiotika verabreicht werden, kommen unterstützend und in leichteren Fällen unter Umständen alleinig zur topischen, symptomatischen Behandlung oftmals Antiseptika und entzündungshemmende Stoffe (so z. B. Antiphlogistika, antiinflammatorisch wirkende Mittel, Lokalantibiotika etc.) zum Einsatz, welche beispielsweise in Form von Rachenspülungen, Sprays oder Lutschtabletten verabreicht werden können. Diese Maßnahmen sind meist nicht ausreichend effizient und nachhaltig.

Die DE 20 2004 016 646 U1 betrifft pharmazeutische Zusammensetzungen in Form einer festen Dosierung zum Lutschen, insbesondere Lutschtabletten, zur topischen Behandlung von entzündlichen Erkrankungen des Mund- und Rachenraums, wobei die Zusammensetzung den Wirkstoff Octenidin als Antiseptikum enthält.

Weiterhin betrifft die WO 2007/110871 A2 ein Verfahren zur Behandlung von Halsschmerzen bzw. Halsentzündungen, wobei in diesem Zusammenhang eine pharmazeutische Zusammensetzung in den Rachen appliziert wird. Die Zusammensetzung weist einen öligen Exzipienten und einen pharmazeutisch aktiven Inhaltsstoff auf und wird vorzugsweise in Form eines Sprays appliziert.

Der vorliegenden Erfindung liegt daher die Aufgabe zugrunde, eine Zusammensetzung, insbesondere eine pharmazeutische Zusammensetzung, bereitzustellen, welche die zuvor geschilderten Nachteile des Standes der Technik zumindest weitgehend vermeidet oder aber wenigstens abschwächt.

Im Speziellen liegt der vorliegenden Erfindung die Aufgabe zugrunde, eine Zusammensetzung bereitzustellen, welche sich zur effizienten topischen Behandlung von Heiserkeit bzw. Halsschmerzen bzw. entzündlichen Erkrankungen des Mund- und Rachenraums eignet.

Darüber hinaus liegt der vorliegenden Erfindung die Aufgabe zugrunde, eine Zusammensetzung bereitzustellen, welche im Rahmen der Behandlung von Heiserkeit bzw. entzündlichen Erkrankungen des Mund- und Rachenraums eine hervorragende und lang andauernde Linderung der Schmerz- und Entzündungssymptomatik erlaubt und zudem dem Symptom der Mundtrockenheit entgegenwirkt.

Zur Lösung der zuvor geschilderten Aufgabe schlägt die vorliegende Erfindung eine Zusammensetzung nach Anspruch 1 vor; weitere vorteilhafte Ausgestaltungen sind Gegenstand der diesbezüglich abhängigen Ansprüche.

Es versteht sich von selbst, dass im Folgenden besondere Ausgestaltungen, Ausführungsformen oder dergleichen, welche nur im Zusammenhang mit einem Erfindungsaspekt beschrieben sind, auch in Bezug auf die anderen Erfindungsaspekte entsprechend gelten, ohne dass dies einer ausdrücklichen Erwähnung bedarf.

Weiterhin ist bei allen nachstehend genannten relativen bzw. prozentualen, insbesondere gewichtsbezogenen Mengenangaben zu beachten, dass diese im Rahmen der vorliegenden Erfindung vom Fachmann derart auszuwählen sind, dass in der Summe der jeweiligen Inhaltsstoffe, Wirkstoffe, Zusatz- bzw. Hilfsstoffe oder dergleichen stets 100 % resultieren. Dies versteht sich für den Fachmann aber von selbst.

Im Übrigen gilt, dass der Fachmann anwendungsbezogen oder einzelfallbedingt von den nachfolgend aufgeführten Zahlen-, Bereichs- oder Mengenangaben abweichen kann, ohne dass er den Rahmen der vorliegenden Erfindung verlässt.

Zudem gilt, dass alle im Folgenden genannten Parameterangaben oder dergleichen grundsätzlich mit genormten oder explizit angegebenen Bestimmungsverfahren oder aber mit dem Fachmann an sich geläufigen Bestimmungsmethoden bestimmt bzw. ermittelt werden können.

Gegenstand der vorliegenden Erfindung ist somit - gemäß einem **e r s t e n** Erfindungsaspekt - eine Zusammensetzung, insbesondere pharmazeutische Zusammensetzung, vorzugsweise in Form einer festen oder flüssigen, insbesondere festen Dosierung, welche zur topischen Behandlung von Heiserkeit oder Halsschmerzen bzw. entzündlichen Erkrankungen des Mund-und Rachenraums geeignet ist,
wobei die Zusammensetzung - in Kombination und jeweils in wirksamen, insbesondere pharmazeutisch wirksamen Mengen -
(a) mindestens ein virustatisches und/oder antiviral wirksames Polysaccharid, ausgewählt aus Carrageenen und/oder deren physiologisch verträglichen Salzen, Galactansulfaten und/oder deren physiologisch verträglichen Salzen und Polyuroniden und/oder deren physiologisch verträglichen Salzen ("Komponente (a)");
(b) Polyhexanid als Oberflächendesinfektionsmittel, ("Komponente (b)"); sowie
(c) Hyaluronsäure oder deren Salze ("Komponente (c)");
aufweist.

Die Anmelderin hat nunmehr in überraschender Weise herausgefunden, dass eine Zusammensetzung, vorzugsweise eine pharmazeutische Zusammensetzung, welche eine Wirkstoffkombination auf Basis mindestens eines virustatischen bzw. antiviral wirksamen Polysaccharids mit Polyhexanid als Oberflächendesinfektionsmittel sowie Hyaluronsäure aufweist, eine hervorragende Linderung von Heiserkeit oder Halsschmerzen sowie der damit einhergehenden Schmerz- und Entzündungssymptomatik verschafft und gleichzeitig eine effektive Linderung von Mundtrockenheit bewirkt. In diesem Zusammenhang hat sich insbesondere gezeigt, dass die Wirkung der Einzelwirkstoffe in der erfindungsgemäßen Kombination über die der Einzelwirkstoffe bei alleinigem Einsatz in signifikanter Weise hinausgeht, was als Indiz für einen synergistischen Effekt zu werten ist.

Die Begriffe "pharmazeutische Zusammensetzung", "pharmazeutische Zubereitung" oder dergleichen, wie sie im Rahmen der vorliegenden Erfindung verwendet werden, sind sehr umfassend zu verstehen und bezeichnen nicht nur pharmazeutische Präparate bzw. Pharmazeutika, sondern auch sogenannte Medizinprodukte, homöopathische Mittel, Nahrungsergänzungsmittel oder dergleichen.

Im Rahmen der vorliegenden Erfindung werden unter dem Begriff "Polysaccharide" ("Komponente (a)") makromolekulare Kohlenhydrate, deren Moleküle aus einer Zahl von mindestens > 10 glykosidisch miteinander verknüpften Monosaccharid-Molekülen bestehen, zusammengefasst. Zu den Polysacchariden gehören zahlreiche Biopolymere bzw. Polymere biogenen Ursprungs, welche äußerst variable chemischen bzw. pharmakologische Eigenschaften besitzen können. Polysaccharide dienen vorwiegend als Reservestoffe und liegen üblicherweise als Homopolymere bzw. Homoglykane vor, welche jeweils eine Art von Monosaccharid zugrundeliegt. Zudem existieren Polysaccharide, welche unter Gelbildung große Mengen Wasser, binden können und demzufolge als sogenannte Schleimstoffe bezeichnet werden. Zu den Schleimstoffen gehörende Polysaccharide liegen meistens als Heteropolymere oder Heteroglykane auf Basis verschiedenartiger Monomereinheiten vor. Bekannte Schleimstoffe sind Pektine, Mannane, Galaktane, Xylane, Hyaluronsäure bzw. Glykosaminoglykane sowie zahlreiche Polymere auf Basis von sulfonierten Galaktanen, wie beispielsweise Carrageene, sowie Uronsäuren. Für die erfindungsgemäße Zusammensetzung hat sich der Einsatz von solchen gelbildenden Polymeren als besonders vorteilhaft erwiesen, da diese eine besonders starke Affinität zur Mundschleimhaut bzw. Rachenschleimhaut zeigen und sozusagen einen Schutzfilm bilden, welcher die Schleimhaut einerseits vor dem Eindringen von Krankheitserregern, wie beispielsweise Erkältungsviren, und andererseits einem Flüssigkeitsverlust bzw. Mundtrockenheit vorbeugt. Darüber hinaus existieren auch Polysaccharide, insbesondere Schleimstoffe, welche über antivirale Eigenschaften verfügen. Der Einsatz derartiger Polysaccharide ist für die erfindungsgemäße Zusammensetzung vorgesehen.

Für weitergehende Einzelheiten zum Begriff der "Polysaccharide" kann verwiesen werden auf Römpp, Chemielexikon, 10. Auflage, Georg Thieme Verlag, Stuttgart / New York, Seite 3503, Stichwort: "Polysaccharide", sowie auf die darin referierte Literatur, deren diesbezüglicher Inhalt hiermit vollumfänglich durch Bezugnahme eingeschlossen ist.

Unter den im Rahmen der vorliegenden Erfindung eingesetzten Oberflächendesinfektionsmitteln ("Komponente (b)") werden insbesondere solche Desinfektionsmittel verstanden, welche für die Anwendung auf der Haut bzw. Schleimhaut geeignet sind. Die dem jeweiligen Desinfektionsmittel zugrundeliegenden Wirkstoffe können insbesondere auf Oxidationsmitteln, wie Wasserstoffperoxid oder Iod, sowie Alkoholen, Phenolen, Stickstoffverbindungen, insbesondere quartären Ammoniumsalzen, sowie Detergenzien basieren. Üblicherweise besitzen derartige Desinfektionsmittel eine bakterizide, fungizide sowie viruzide Wirkweise, so dass ein zumindest im Wesentlichen vollumfänglicher Schutz vor pathogenen Erregern gewährleistet ist.

Bei den erfindungsgemäß verwendeten vorzugsweise sauren Glykosaminoglykanen ("Komponente (c)") handelt es sich um lineare Mucopolysaccharide, welche aus sich wiederholenden Sacchariden gebildet werden. Im Allgemeinen bestehen die Saccharid-Einheiten aus einer Uronsäure, wie insbesondere Glucuronsäure, aber auch Iduronsäure oder Uronsäure, welche 1,3-glykosidisch mit einem Aminozucker, wie N-Acetylglucosamin, verknüpft sind. Die Saccharid-Einheiten zur Ausbildung der Kette als solcher wiederum sind 1,4-glykosidisch miteinander verbunden. Insbesondere kann es auch vorgesehen sein, dass die Glykosaminoglykane mit Schwefelsäure oder Essigsäure weiter verestert sind. Die in Rede stehenden Glykosaminoglykane liegen häufig in Assoziation mit biologischen Makromolekülen vor und können beispielsweise in großer Zahl kovalent an Proteine gebunden vorkommen. Auf diese Weise können sie das Gerüst vieler faserbildender Stoffe, wie z.B. Fibrillin, im Körper ausbilden. Ein erfindungsrelevanter Vorteil bzw. eine erfindungsrelevante Eigenschaft der Glykosaminoglykane liegt insbesondere in ihrer Fähigkeit, in großen Mengen Wasser aufnehmen zu können, so dass bei topischer Applikation ein Austrocknen der Haut bzw. Schleimhaut verhindert wird.

Im Rahmen der vorliegenden Erfindung wird als Glykosaminoglykan Hyaluronsäure oder deren Salze eingesetzt. Hyaluronsäure stellt im menschlichen bzw. tierischen Körper einen wichtigen Bestandteil des Bindegewebes dar und spielt darüber hinaus eine Rolle bei der Zellproliferation und Zellmigration. Im Rahmen der vorliegenden Erfindung hat sich insbesondere die zellproliferative Wirkung in Bezug auf eine schnelle Regenerierung des Gewebes als vorteilhaft erwiesen. Weiterhin besitzt auch Hyaluronsäure die Fähigkeit, äußerst große Mengen an Wasser zu binden: So können mit 1 g Hyaluronsäure bis zu 6 Liter Wasser gebunden werden. Durch die Bindung großer Mengen an Wasser kann einer Austrocknung der Haut bzw. Schleimhaut in effizienter Weise vorgebeugt werden, so dass eine beschleunigte Regeneration des Gewebes ermöglicht wird und die Haut bzw. Schleimhaut darüber hinaus elastisch bleibt.

Chemisch gesehen handelt es sich bei Hyaluronsäure um eine makromolekulare Kette aus Disacchariden auf Basis von D-Glucuronsäure und N-Acetyl-D-Glucosamin, welche über eine beta-1,3-glykosidische Bindung miteinander verknüpft sind. Die Disaccharide wiederum sind über eine beta-1,4-glykosidische Bindung zu einer polymeren Kette miteinander verbunden. Im Allgemeinen besteht ein Hyaluronsäure-Polymer aus 250 bis 50.000 Disaccharideinheiten.

Die Anmelderin hat nämlich im Rahmen der vorliegenden Erfindung überraschenderweise herausgefunden, dass sich durch die synergistische Kombination von mindestens einem virustatischen bzw. antiviral wirksamen Polysaccharid einerseits sowie mindestens einem Oberflächendesinfektionsmittel andererseits und zusätzlich mindestens einem Glykosaminoglykan Heiserkeit als solche und darüber hinaus auch die oftmals der Heiserkeit zugrundeliegenden entzündlichen Erkrankungen des Mund- und Rachenraums (z. B. Halsschmerzen), insbesondere die damit einhergehende Schmerz- und Entzündungssymptomatik und das Symptom der Mundtrockenheit, besonders kurzfristig lindern lassen.

Die vorliegende Erfindung weist zahlreiche weitere Vorteile und Besonderheiten auf, welche sie gegenüber dem Stand der Technik auszeichnen und nachfolgend ausführlich geschildert sind:
Die eingesetzten Polysaccharide zeichnen sich durch eine gute Adsorption bzw. Anheftung an die Mundschleimhaut aus, was die Ausbildung eines schützenden Films auf der Schleimhaut ermöglicht. Ein derartiger Film verhindert einerseits das Eindringen von Krankheitserregern, wie beispielsweise Viren und Bakterien, und beugt durch seine stark wasserbindenden Eigenschaften andererseits Mundtrockenheit bzw. dem Austrocknen der Schleimhäute im Mund- und Rachenraum vor. Weiterhin zeichnen sich die eingesetzten Polysaccharide durch ihre hervorragende Verträglichkeit aus, da sie üblicherweise zumindest im Wesentlichen nebenwirkungsfrei sind. Durch den Einsatz antiviral wirksamer Polysaccharide kann zudem die Schutzwirkung auf Basis der Ausbildung eines Films noch weitergehend verstärkt werden.

Der Einsatz mindestens eines Oberflächendesinfektionsmittels hat sich im Rahmen der vorliegenden Erfindung dahingehend als besonders vorteilhaft erwiesen, dass in Kombination mit der Barrierewirkung der eingesetzten Polysaccharide ein vollumfänglicher Schutz gegenüber den üblichen Bakterien und Viren, welche besonders häufig im Zusammenhang mit grippalen Infekten und Erkältungskrankheiten vorliegen und somit häufig mit Heiserkeit oder Halsschmerzen assoziiert sind, gewährleistet wird.

Insgesamt ergänzen sich somit die erfindungsgemäß eingesetzten Polysaccharide einerseits und das mindestens eine Oberflächendesinfektionsmittel andererseits in synergistischer Weise, da die Wirkungen der beiden Komponenten in kombiniertem Einsatz über die Wirkung der jeweiligen Einzelwirkstoffe bei alleinigem Einsatz hinausgeht. Insbesondere in Bezug auf den Schutz vor Krankheitserregern bzw. pathogenen Keimen findet eine Wirkverstärkung statt, da durch den schützenden Film einerseits eine Barriere gegenüber den Erregern gebildet wird und durch das Oberflächendesinfektionsmittel darüber hinaus eine gezielte Abtötung derartiger Erreger stattfindet. Die vorgenannten Effekte werden durch den zusätzlichen Einsatz mindestens eines vorzugsweise sauren Glykosaminoglykans, insbesondere Hyaluronsäure, noch weiter verstärkt, da auch saure Glykosaminoglykane, insbesondere Hyaluronsäure, stark wasserbindende Eigenschaften aufweisen und somit insbesondere die Bildung eines Schutzfilms sowie die Vorbeugung bzw. Linderung von Mundtrockenheit weitergehend verstärken.

Darüber hinaus zeichnet sich die erfindungsgemäße Zusammensetzung gegenüber den aus dem Stand der Technik bekannten Methoden zur Linderung von Heiserkeit oder Halsschmerzen bzw. entzündlichen Erkrankungen im Mund- und Rachenraum durch eine schnell eintretende und dennoch äußerst lang anhaltende Wirkung aus. Auch einige Stunden nach Applikation der Zusammensetzung besteht weiterhin eine gute Benetzung der Schleimhäute und darüber hinaus eine für den Patienten angenehme Linderung der Schmerzsymptomatik.

Weiterhin bewirkt die erfindungsgemäße Zusammensetzung insgesamt eine äußerst schnelle Regeneration bzw. Wiederherstellung der gestörten Phonation, d. h. die stimmlichen Beeinträchtigungen, insbesondere die belegte, raue oder heisere Stimme, werden schnell geheilt. Auch Halsschmerzen oder andere entzündliche Erkrankungen des Mund- und Rachenraums lassen sich mit der erfindungsgemäßen Zusammensetzung in effizienter Weise behandeln.

Darüber hinaus zeichnet sich die erfindungsgemäße Zusammensetzung durch eine hervorragende Verträglichkeit aus, da sie zumindest im Wesentlichen frei von etwaigen Nebenwirkungen ist. Aufgrund der guten Verträglichkeit ist die erfindungsgemäße Zusammensetzung somit auch für einen verhältnismäßig lang andauernden Gebrauch bzw. Einsatz sowie zur Verwendung bei Kindern geeignet.

Schließlich wird im Zusammenhang mit den zuvor beschriebenen Vorteilen und Besonderheiten bereits an dieser Stelle auf die von der Anmelderin durchgeführten Wirksamkeitsstudien verwiesen, welche die vorgenannten Effekte in eindrucksvoller Weise belegen und nachfolgend noch detailliert beschrieben sind.

Die erfindungsgemäße Zusammensetzung kann in vielfältiger Art und Weise ausgestaltet sein. Bevorzugte Ausführungsformen sind im Folgenden zum besseren Verständnis beschrieben.

Um eine gute Anheftung des Polysaccharids an die Mundschleimhaut bzw. Rachenschleimhaut zu erzielen, hat es sich als vorteilhaft erwiesen, Polysaccharide mit definierten gewichtsmittleren Molekulargewichten einzusetzen. Gemäß einer bevorzugten Ausführungsform der vorliegenden Erfindung kann es somit vorgesehen sein, dass die Komponente (a) ein gewichtsmittleres Molekulargewicht im Bereich von 5.000 bis 10.000.000 Da, insbesondere im Bereich von 15.000 bis 7.500.000 Da, vorzugsweise im Bereich von 20.000 bis 5.000.000 Da, bevorzugt im Bereich von 30.000 bis 3.000.000 Da, aufweist. Die Bestimmung des Molekulargewichts erfolgt vorzugsweise mittels Gelpermeationschromatographie (GPC), vorzugsweise nach DIN 55672.

Um darüber hinaus eine besonders gute Verträglichkeit sicherzustellen, hat es sich insbesondere als vorteilhaft erwiesen, wenn die Komponente (a) ein Biopolymer bzw. ein Polymer biogenen Ursprungs ist.

Wie bereits zuvor erwähnt, können Polysaccharide sowohl als Homopolysaccharide bzw. Homoglykane als auch als Heteropolysaccharide bzw. Heteroglykane vorliegen. Erfindungsgemäß kann es somit vorgesehen sein, dass die Komponente (a) aus Homopolysacchariden und/oder Heteropolysacchariden ausgewählt ist. Üblicherweise handelt es sich bei Polysacchariden mit gelbildenden Eigenschaften um Heteropolysaccharide, was sich vor dem Hintergrund einer guten Adsorption bzw. Anheftung an die Mundschleimhaut im Rahmen der vorliegenden Erfindung als vorteilhaft erwiesen hat. Bevorzugt ist somit im Rahmen der vorliegenden Erfindung der Einsatz von Heteropolysacchariden.

Eine besonders gute Adsorption an die Mundschleimhaut und somit ein hervorragender Schutz der Schleimhaut vor Austrocknung einerseits sowie eindringenden Keimen andererseits kann erzielt werden, wenn die Komponente (a) aus Schleimstoffen, insbesondere Galactansulfaten und/oder deren Derivaten und/oder deren physiologisch verträglichen Salzen und/oder Polyuroniden und/oder deren Derivaten und/oder deren physiologisch verträglichen Salzen ausgewählt ist.

Gemäß einer bevorzugten Ausführungsform der vorliegenden Erfindung kann es in diesem Zusammenhang vorgesehen sein, dass die Komponente (a), insbesondere das Polysaccharid, vorzugsweise als Schleimstoff, bevorzugt in Form einer Droge, insbesondere in Form zerkleinerter oder pulverisierter oder extrahierter Algen, Pflanzenteile oder Pflanzenbestandteile, bereitgestellt ist.

Gemäß einer weiteren bevorzugten Ausführungsform der erfindungsgemäßen Zusammensetzung kann es vorgesehen sein, dass die Komponente (a), insbesondere das Polysaccharid, vorzugsweise der Schleimstoff, in Form eines Extraktes, insbesondere eines Trockenextraktes, bereitgestellt ist. Vorzugsweise wird der Extrakt ausgehend von einem wässrigen, alkoholischen oder wässrig-alkoholischen Extrakt, bevorzugt einem wässrigen Extrakt, erhalten.

In diesem Zusammenhang lässt sich die Wirksamkeit der erfindungsgemäßen Zusammensetzung noch weitergehend steigern, wenn der Extrakt, vorzugsweise der Trockenextrakt, ein Droge/Extrakt-Verhältnis (DEV), insbesondere berechnet als gewichtsbezogenes Verhältnis von eingesetzter Ausgangsmenge an Droge zu erhaltenem Extrakt, im Bereich von 0,1 : 1 bis 50 : 1, insbesondere im Bereich von 0,5 : 1 bis 25 : 1, vorzugsweise im Bereich von 2 : 1 bis 20 : 1, besonders bevorzugt im Bereich von 5 : 1 bis 10 : 1, aufweist.

Das sogenannte Droge/Extrakt-Verhältnis (DEV) charakterisiert das (gewichtsbezogene) Verhältnis von eingesetzter Ausgangsdroge zu erhaltenem Extrakt. Das Droge/Extrakt-Verhältnis (DEV) gibt also an, aus welcher gewichtsbezogenen Menge an eingesetzter Droge (z. B. Rotalgen) welche gewichtsbezogene Menge an Extrakt gewonnen ist. Ein Droge/Extrakt-Verhältnis beispielsweise von 10 : 1 bedeutet, dass aus zehn Gewichtsteilen Droge ein Gewichtsteil Extrakt gewonnen wurde. Das Droge/Extrakt-Verhältnis gibt also an, wieviele Gewichtsteile einer Arzneidroge für die Herstellung des gewichtsbezogenen Extraktäquivalents benötigt werden.

Die Verwendung von Extrakten mit definierten Droge/Extrakt-Verhältnis ist insbesondere vor dem Hintergrund relevant, dass die Qualität des Extraktes einen entscheidenden Einfluss auf die Gesamtqualität der pharmazeutischen Zusammensetzung hat. Ziel ist es dabei zudem, den Extrakt hinsichtlich der Wirkstoffkonzentration zu standardisieren, so dass auch die finale pharmazeutische Zubereitung qualitativ und quantitativ mit konstant guten Werten bezüglich der Wirk- und Inhaltsstoffe bereitgestellt werden kann.

Was die Komponente (a) bzw. das Polysaccharid bzw. den Schleimstoff weiterhin anbelangt, so ist es im Rahmen der vorliegenden Erfindung bevorzugt, wenn dieser erhältlich ist aus Isländischem Moos (*Lichen islandicus*), Eibisch *(Althaea officinalis L.),* Spitzwegerich *(Plantago lanceolata L.*), Malve *(Malva sylvestris L.* und *M. neglecta WALLR.),* Boxhornklee (*Trigonella foenum-graecum L.*), Salep, Quitte *(Cydonia oblonga MILL.*) und Algen, vorzugsweise Rotalgen, insbesondere Rotalgen-Spezies der Gattungen *Chondrus, Euchema* und/oder *Gigartina,* sowie deren Kombinationen.

Isländisches Moos ist der deutsche Name für *Lichen islandicus* oder *Cetraria islandica (Parmeliaceae),* einer Flechte - entgegen dem deutschen Namen kein Moos -, die aus nördlichen Ländern, wie beispielsweise von Island, Norwegen und Schweden, exportiert wird. Isländisches Moos im eigentlichen Sinne besteht aus dem getrockneten Thallus von *Cetraria islandica* sowie dessen Zubereitungen. Die Droge enthält unter anderem Schleimstoffe und Bitterstoffe sowie bitterschmeckende Flechtensäuren. Aus der gepulverten Flechte lösen sich etwa 60 Gew.-% beim Kochen mit stark verdünnter Natriumhydrogencarbonatlösung, und beim Abkühlen der Lösung entsteht eine Gallerte. Der Extrakt besteht aus einem Polysaccharidgemisch von Lichenin und Isolichenin, einer Reihe von bitterschmeckenden Flechtensäuren (Fumarprotocetrar-, Protocetrar- und Cetrarsäure) sowie Protolichesterinsäure, die sich bei der Aufarbeitung in Lichesterinsäure umwandelt, und Usninsäure als antibiotisch wirkendem Flechtenfarbstoff. Als Schleimdroge besitzt Isländisches Moos reizlindernde Eigenschaften, es wirkt ebenso antimikrobiell. Der Bitterstoffgehalt begründet seine Anwendung bei Appetitlosigkeit. Isländisches Moos wird beispielsweise bei Katarrhen und Durchfall, als Bittertonikum, äußerlich bei schlecht heilenden Wunden, bei kosmetischen Präparaten, bei Appetitlosigkeit sowie bei Schleimhautreizungen im Mund- und Rachenraum angewandt. Ein Mund- und Rachendesinfiziens auf Basis von Isländischem Moos wirkt reizlindernd bei trockenem Reizhusten. Das Kaltmazerat und andere bitterschmeckende Zubereitungen der Droge werden zur Behebung der Sub- oder Antazidität eingesetzt. Als Darreichungsformen werden je nach Anwendungsgebiet die zerkleinerte Droge für Aufgüsse sowie andere galenische Zubereitungen bzw. die zerkleinerte Droge vorzugsweise für Kaltmazerate sowie andere bitterschmeckende Zubereitungen zum Einnehmen angewandt. Für weitergehende Einzelheiten zu Isländischem Moos kann beispielsweise auf die folgende Literaturstellen verwiesen werden: Römpp Chemie-Lexikon, 9. Auflage, Band 3, Georg Thieme Verlag, Stuttgart/New York, 1990, Seiten 2055/2056, Stichwort: "Isländisches Moos"; HagerROM 2001, Springer Verlag, Heidelberg, Stichworte: "Cetraria", "Cetraria ericetorum OPIZ", "Cetraria islandica (L.) ACHARIUS" und "Lichen islandicus (Isländisches Moos)"; Monographie "Lichen islandicus (Isländisches Moos)", Bundesanzeiger Nr. 43 vom 2. März 1989; H. Wagner, "Pharmazeutische Biologie - Drogen und ihre Inhaltsstoffe", Gustav Fischer Verlag, Stuttgart/New York, 1985, Seite 281, Stichwort: "Cetrariae Lichen (= Lichen islandicus - Isländisches Moos)".

Der Eibisch (*Althaea officinalis L.),* eingesetzt in Form der Eibischwurzel, -blätter und -blüten, wächst auf salzhaltigen und feuchten Böden Mittel-, Ost- und Südosteuropas. Die im Herbst geernteten, von der holzigen Hauptwurzel, von Wurzelfasern und Rindenschichten befreiten und bei 35 °C getrockneten Wurzelzweige und Nebenwurzeln kommen geschält oder ungeschält in den Handel. Die Braunfärbung der geschälte Droge bedeutet eine Qualitätsminderung; ein nachträgliches "Schönen" mit Sulfitlösung ist unzulässig. Weiterhin kommen die vor oder während der Blüte gesammelten, etwa 10 cm langen, etwa 8 cm breiten, drei- bis fünflappigen, graufilzig behaarten getrockneten Blätter und die im Juli/August gesammelten, fleischfarbenen getrockneten Kronblätter zur Anwendung. Der Eibisch ist eine mehrjährige, etwa 1 m hohe Staude, die generativ oder vegetativ vermehrt wird. Die im Spätherbst geerntete Wurzel enthält bis zu 15 % Schleimstoffe. Im Frühjahr und Sommer liegt der Schleimgehalt bei 5 bis 6 %. Der Schleimgehalt der Blatt- und Blütendroge beträgt 6 bis 9 %. Der Schleim ist in der Wurzel in bestimmten Schleimzellen des Rinden- und Holzparenchyms lokalisiert. Er besteht aus Galacturonorhamnanen, Glucanen und Arabinogalactanen. Wässrige Auszüge aus der Wurzel sollen durch Mazeration bei Zimmertemperatur hergestellt werden, damit die reichlich vorhandene Stärke nicht durch Quellung störend wirkt. Eibischdrogen werden für Teezubereitungen oder zur Herstellung von Sirupus Althaeae verwendet. Das Hauptanwendungsgebiet sind entzündliche Reizzustände des Rachenraums. Äußerlich kommt der Eibisch zu erweichenden Umschlägen, Bädern und Kataplasmen zur Anwendung.

Der Spitzwegerich (*Plantago lanceolata L.*) ist in ganz Europa sowie in Nord-und Mittelasien verbreitet. Die Droge stammt von wild wachsenden Pflanzen und Kulturen vor allem Südosteuropas. Die getrockneten, oliv- bis bräunlichgrün gefärbten, lanzettlichen Blattspreiten mit etwa drei bis sieben parallel verlaufenden Nerven können verwendet werden. Die Herba-Droge besteht aus den getrockneten Blättern, Stengeln und ganzen Blüten. Neben dem Schleim enthält der Spitzwegerich als weitere Inhaltsstoffe Tannine, das Iridoidglykosid Aucubin (1,9 bis 2,4 %), das für die Dunkelfärbung einer nicht sorgfältig getrockneten Droge verantwortlich ist und zur Identitätsprüfung herangezogen wird, ferner das Senföl Sulforaphen. Der Spitzwegerich, insbesondere das Spitzwegerichblätter-Kraut, findet insbesondere Anwendung in Form von Tees und Sirupen bei Entzündungen des Mund- und Rachenraums.

Die Malve (*Malva sylvestris L.* und *M. neglecta WALLR.),* insbesondere eingesetzt in Form von Malvenblüten und -blättern, ist in Europa, Kleinasien, im Mittelmeergebiet und in Vorderindien heimisch. Zur Anwendung kommen die zur Blütezeit gesammelten, rosa-violett gefärbten Blüten bzw. die durch das Trocknen stark eingeschrumpften, gefalteten Blätter, die zumeist in Paketform in den Handel kommen. Die Malve ist ein zwei- bis mehrjähriges Kraut. Der Schleimgehalt von Blüten und Blättern liegt bei 6 bis 8 %. Bei der Hydrolyse liefert der Schleim Glucose, Arabinose, Rhamnose und Galactose. Malvenblüten werden zu Gurgelwässern und Bädern und zusammen mit den Blättern in Teemischungen als Expectorans verwendet.

Der Bockshornklee *(Trigonella foenum-graecum L.),* insbesondere eingesetzt in Form des Bockshornkleesamens, ist im gesamten Mittelmeergebiet, sowie in Osteuropa, Indien und China beheimatet. Der Hauptdrogenimport stammt aus den Kulturen Indiens und Marokkos. Zur Anwendung kommen die braunrötlich gefärbten, vierseitigen und rautenförmigen, etwa 5 mm langen und etwa 3 mm breiten, flachgedrückten, sehr harten Samen, die durch eine Furche charakterisiert sind. Die Droge enthält außer fettem Öl 20 bis 30 % Schleim, Trigonellin, Nicotinsäureamid, Cholin, Bitterstoffe und Saponine. Die Herstellung des Schleims erfolgt aus dem gepulvertem Samen. Das Pulver wird angewendet äußerlich zu Umschlägen bei Furunkeln, Geschwüren und Drüsenschwellungen, innerlich als Expectorans und Roborans.

Salep bzw. Salep Tuber, insbesondere eingesetzt in Form der Salepknolle, ist von verschiedenen Orchideen-Arten abgeleitet, besonders *Orchis mascula L., Orchis morio L., Orchis militaris L., Anacamptis pyramidalis L. RICH.* und *Platanthera bifolia L. RICH.* Zur Anwendung kommen die etwa 4 cm langen bis etwa 3 cm dicken, runzeligen, harten, bräunlichen Knollen, die nach der Ernte von der Korbschicht befreit, mit siedendem Wasser abgebrüht und bei künstlicher Wärme getrocknet werden. Salepschleim, der bis zu 50 % vorwiegend aus Glucomannan bzw. Glucan besteht, wird hauptsächlich in der Kinderheilpraxis als Antidiarrhoikum verwendet.

Die Quitte *(Cydonia oblonga MILL.*) ist im südöstlichen Arabien heimisch. Hautdrogenimporte kommen aus Spanien, Portugal und Persien. Zur Anwendung kommen die reifen, getrockneten, rot-braun bis braun-violett gefärbten, etwas abgeplatteten Samen der Quittenscheinfrucht. Die Samen sind außen zum Teil mit einer eingetrockneten Schleimkruste versehen und oft miteinander verklebt. Sie besitzen einen schwachen Bittermandelgeschmack. In der Epidermis findet sich ca. 22 % Schleim, der zum größten Teil wasserlöslich ist. Die Zuckerkomponenten sind Arabinose, Xylose und Uronsäure, die zum Teil methyliert ist. Die Droge kommt nur unzerkleinert zur Anwendung. Der Quittenschleim wird äußerlich bei Lippen- und Brustwarzenrhagaden, bei Verbrennungen und Dekubitus in Salben- oder Cremeform verwendet.

Unter der Bezeichnung Algen werden im weiteren Sinne im Wasser lebende, eukaryotische, pflanzenartige Lebewesen bezeichnet, welche Photosynthese betreiben, jedoch den eigentlichen Pflanzen zugerechnet werden. Algen zeichnen sich durch einen besonders vielfältigen Stoffwechsel aus, welcher unter anderem die Synthese zahlreicher Polysaccharide vorsieht. Bei den von Algen synthetisierten Polysacchariden handelt es sich insbesondere um Schleimstoffe und Verdickungsmittel, welche bislang vor allem in der Lebensmittel- sowie Kosmetikindustrie Anwendung finden. Insbesondere Rotalgen (*Rhodophyta*) zeichnen sich durch ihre Fähigkeit zur Synthese von Polymeren, wie Agar auf Basis von Agarose, sulfoniertem Agaropektin sowie Carrageen auf Basis von sulfonierter Galaktose und 3,6-Anhydrogalaktose, aus. Sowohl Agar als auch Carrageen bilden bei Kontakt mit Wasser aufgrund ihrer Fähigkeit, Wasser in großen Mengen zu binden, eine gallertartige Masse bzw. einen sogenannten Schleim.

Gemäß einer besonders bevorzugten Ausführungsform der vorliegenden Erfindung kann es vorgesehen sein, dass die Komponente (a), insbesondere das Polysaccharid, vorzugsweise der Schleimstoff, aus den Carrageenen und/oder deren Derivaten und/oder deren physiologisch verträglichen Salzen, insbesondere alpha-, iota-, gamma-, kappa- und/oder lambda-Carrageenen und/oder deren Derivaten und/oder deren physiologisch verträglichen Salzen, vorzugsweise iota-Carrageen und/oder dessen Derivaten und/oder physiologisch verträglichen Salzen, ausgewählt ist.

Gleichermaßen kann es vorgesehen sein, dass die Komponente (a), insbesondere das Polysaccharid, vorzugsweise der Schleimstoff, ein Heteropolymer auf Basis von alpha-, iota-, gamma-, kappa- und/oder lambda-Carrageen und/oder deren Derivaten und/oder deren physiologisch verträglichen Salzen, insbesondere auf Basis von iota- und kappa-Carrageenen und/oder deren Derivaten und/oder deren physiologisch verträglichen Salzen, ist.

Im Rahmen der vorliegenden Erfindung wird unter dem Begriff "Carrageen" bzw. "Carrageenan" eine Sammelbezeichnung für eine Gruppe langkettiger Polysaccharide verstanden, welche insbesondere in Zellen verschiedener Rotalgenarten vorkommen bzw. synthetisiert werden. Bei Carrageenen handelt es sich um lineare anionische Hydrokolloide, wobei je nach chemischer Struktur verschiedene Carrageentypen unterschieden werden. Aufgrund der jeweiligen Struktur ergeben sich für die verschiedenen Typen auch unterschiedliche Eigenschaften. Die Zuordnung erfolgt in erster Linie anhand des Anteils an Galaktose und 3,6-Anhydrogalaktose sowie der Anzahl an Sulfatgruppen. Zur Herstellung von Carrageen aus Rotalgen werden diese zunächst gewaschen und in alkalischer Lösung gekocht. Anschließend wird die Lösung filtriert, um die Zelltrümmer bzw. Algenbestandteile von dem Polymer zu separieren. Zur Gewinnung des Carrageens aus der Lösung wird das Carrageen schließlich entweder mittels Alkohol ausgefällt oder mittels Kaliumchlorid geliert und anschließend abgepresst. Das gewonnene Carrageen kann anschließend getrocknet, vermahlen und je nach Einsatzzweck weiterverarbeitet werden. Von besonderem kommerziellen Interesse sind insbesondere kappa-, iota- und lambda-Carrageen. Dennoch können alternativ auch andere Carrageentypen eingesetzt werden, welche dann insbesondere als Vorstufen für kappa-, iota- und lambda-Carrageen dienen.

Im Rahmen der vorliegenden Erfindung hat sich überraschenderweise gezeigt, dass durch den Einsatz von Carrageen als Polysaccharid einerseits hervorragende antivirale Wirkungen bei Anwendung auf der Schleimhaut im Hals- und Rachenraum erzielt werden können; andererseits bildet Carrageen aufgrund seiner hydrokolloidartigen Eigenschaften einen hervorragenden Schutzfilm auf der Mund- und Rachenschleimhaut, um Mundtrockenheit bzw. einem Austrocknen der Schleimhäute in effizienter Weise vorzubeugen und das Eindringen von Krankheitserregern verhindern.

Im Zusammenhang mit dem mindestens einen Polysaccharid bzw. der Komponente (a) hat es sich zudem als vorteilhaft erwiesen, die Substanz in definierten Mengen einzusetzen. Als besonders vorteilhaft in Bezug auf die Wirksamkeit hat es sich erwiesen, wenn die Zusammensetzung die Komponente (a) in einer relativen Menge im Bereich von 0,1 bis 40 Gew.-%, insbesondere im Bereich von 0,5 bis 30 Gew.-%, vorzugsweise im Bereich von 1 bis 25 Gew.-%, bevorzugt im Bereich von 2 bis 20 Gew.-%, besonders bevorzugt im Bereich von 3 bis 18 Gew.-%, bezogen auf die Zusammensetzung, enthält.

Diesbezüglich hat es sich als vorteilhaft erwiesen, wenn die Zusammensetzung die Komponente (a) in einer absoluten Menge im Bereich von 0,001 bis 5 g, insbesondere im Bereich von 0,005 bis 4 g, vorzugsweise im Bereich von 0,01 bis 3 g, bevorzugt im Bereich von 0,05 bis 2 g, insbesondere bezogen auf eine Applikatiönseinheit (z. B. Lutschtabletten etc.) und/oder insbesondere auf eine Applikationsmenge, enthält.

Wie zuvor bereits geschildert, ist der Einsatz eines Oberflächendesinfektionsmittels insbesondere vor dem Hintergrund einer Keimzahlreduzierung in Ergänzung zu der Barrierewirkung des Schutzfilms auf Basis von Polysacchariden vorteilhaft. In diesem Zusammenhang ist es vorgesehen, dass die Komponente (b) Polyhexanid ist.

Bei Polyhexanid handelt es sich um ein gängiges Antiseptikum zur Wundbehandlung, bei welchem es sich chemisch gesehen um Polyhexamethylenbiguanid (PHNB) handelt. Vorteilhaft an Polyhexanid ist insbesondere sein breites Wirkungsspektrum, welches auch gegen die schwer zu behandelnden Krankenhauskeime, wie beispielsweise *Staphylococcus aureus,* gerichtet ist. Darüber hinaus wirkt Polyhexanid bereits in äußerst geringen Konzentrationen, da es direkt an die bakterielle Zellwand bindet und diese derart schädigt, dass es zum Zelltod kommen kann. Auf diese Weise wird der Einsatz äußerst geringer Konzentrationen möglich, was das Auftreten von Nebenwirkungen minimiert. Darüber hinaus zeichnet sich Polyhexanid durch eine geringe systemische Resorption aus, was ebenfalls das Risiko des Auftretens von Nebenwirkungen weitergehend minimiert.

Was die eingesetzten Mengen des Oberflächendesinfektionsmittels anbelangt, so können diese in weiten Bereichen variieren. Es hat sich erfindungsgemäß als vorteilhaft erwiesen, wenn das Oberflächendesinfektionsmittel in einer Menge im Bereich von 0,001 bis 5 Gew.-%, insbesondere im Bereich von 0,01 bis 2,5 Gew.-%, vorzugsweise im Bereich von 0,05 bis 2 Gew.-%, bevorzugt im Bereich von 0,01 bis 1 Gew.-%, besonders bevorzugt im Bereich von 0,05 bis 0,5 Gew.-%, bezogen auf die Zusammensetzung, in der Zusammensetzung enthalten ist.

Weiterhin hat es sich im Rahmen der vorliegenden Erfindung - wie zuvor bereits erwähnt - als besonders vorteilhaft erwiesen, die Wirkung des mindestens einen Polysaccharids durch den zusätzlichen Einsatz eines vorzugsweise sauren Glykosaminoglykans zu unterstützen. Im Rahmen der vorliegenden Erfindung ist es vorgesehen, dass das vorzugsweise Glykosaminoglykan Hyaluronsäure oder deren physiologisch unbedenkliche Salze, vorzugsweise das Natriumsalz der Hyaluronsäure, ist.

Was die eingesetzte Menge der Komponente (c) bzw. des sauren Glykosaminoglykans, insbesondere der Hyaluronsäure bzw. deren Salzen, anbelangt, so ist diese gleichermaßen variabel. Es hat sich als vorteilhaft erwiesen, wenn die Zusammensetzung die Komponente (c) in einer Menge im Bereich von 0,01 bis 10 Gew.-%, insbesondere im Bereich von 0,05 bis 5 Gew.-%, vorzugsweise im Bereich von 0,1 bis 1 Gew.-%. bezogen auf die Zusammensetzung, enthält.

Im Hinblick auf eine Linderung der Schmerzsymptomatik bei Heiserkeit bzw. entzündlichen Erkrankungen des Mund- und Rachenraums (wie z. B. Halsschmerzen) hat es sich zudem als vorteilhaft erwiesen, wenn die Zusammensetzung als weitere Komponente (d) mindestens ein Lokalanästhetikum aufweist.

In diesem Zusammenhang ist es von Vorteil, wenn das Lokalanästhetikum bzw. die Komponente (d) aus der Gruppe von Polidocanol, Benzocain, Lidocain und/oder Ambroxol ausgewählt ist. Besonders gute Ergebnisse werden jedoch erzielt, wenn die Komponente (d) Polidocanol ist.

Bei dem im Rahmen der vorliegenden Erfindung verwendeten Polidocanol handelt es sich gemäß der Definition des Europäischen Arzneibuchs 6.0 um ein Gemisch von Ethern verschiedener Macrogole mit Fettalkoholen, hauptsächlich Laurylalkohol. Synonym wird Polidocanol auch als Hydroxypolyethoxydodecan, Macrogollaurylether oder Lauromacrogol 400 bezeichnet. Aufgrund des lipophilen Dodecylrests und der hydrophilen Etherkette lässt sich Polidocanol gut mit Wasser mischen. Darüber hinaus besitzt Polidocanol oberflächenaktive Eigenschaften. Im Stand der Technik wird Polidocanol im Gebiet der Medizin insbesondere zur Verödung von Hämorrhoiden oder Besenreisern in das betroffene Gewebe injiziert; darüber hinaus kann Polidocanol im Stand der Technik in Hautsalben zur Schmerz-und Juckreizlinderung der äußeren Haut (Lederhaut bzw. Dermis) verwendet werden. Im Rahmen der vorliegenden Erfindung kann überraschenderweise erstmals eine Applikation von Polidocanol auf der empfindlichen Schleimhaut realisiert werden, insbesondere im Zusammenwirken mit den übrigen Komponenten wird in vollkommen überraschender Weise keinerlei Reizung der Schleimhäute beobachtet. Für weitergehende Einzelheiten zum Wirkstoff "Polidocanol" kann verwiesen werden auf Römpp, Chemielexikon, 10. Auflage, Georg Thierne Verlag, Stuttgart / New York, Band 5, Seite 3403, Stichwort: "Polidocanol", sowie auf die darin referierte Literatur, deren diesbezüglicher Inhalt hiermit vollumfänglich durch Bezugnahme eingeschlossen ist.

Die eingesetzte Menge des Lokalanästhetikums kann ebenfalls in weiten Bereichen variieren. Erfindungsgemäß hat es sich als besonders wirkungsvoll erwiesen, wenn die Zusammensetzung die Komponente (d) in einer relativen Menge im Bereich von 0,01 bis 5 Gew.-%, insbesondere im Bereich von 0,05 bis 2,5 Gew.-%, vorzugsweise im Bereich von 0,01 bis 1,5 Gew.-%, bevorzugt im Bereich von 0,1 bis 1 Gew.-%, bezogen auf die pharmazeutische Zusammensetzung, enthält.

In diesem Zusammenhang kann es vorgesehen sein, dass die Zusammensetzung die Komponente (d) in einer absoluten Menge im Bereich von 0,01 bis 500 mg, insbesondere im Bereich von 0,05 bis 250 mg, vorzugsweise im Bereich von 0,1 bis 150 mg, bevorzugt im Bereich von 0,5 bis 50 mg, besonders bevorzugt im Bereich von 1 bis 20 mg, bezogen auf eine Applikationseinheit, enthält.

Darüber hinaus hat es sich als erfindungswesentlich erwiesen, die einzelnen Komponenten in jeweils spezifischen Gewichtsverhältnissen zueinander einzusetzen:
Was das Polysaccharid einerseits sowie das Oberflächendesinfektionsmittel andererseits anbelangt, so ist es erfindungsgemäß von Vorteil, wenn die Zusammensetzung die Komponente (a) und die Komponente (b) in einem gewichtsbezogenen Verhältnis von ([a] : [b]) im Bereich von 1 : 1 bis 1.000 : 1, insbesondere im Bereich von 5 : 1 bis 500 : 1, vorzugsweise im Bereich von 15 : 1 bis 100 : 1, bevorzugt im Bereich von 20 : 1 bis 70 : 1, aufweist.

Darüber hinaus hat es sich als vorteilhaft erwiesen, wenn die erfindungsgemäße Zusammensetzung das mindestens eine Polysaccharid bzw. Komponente (a) einerseits sowie das mindestens eine vorzugweise saure Glykosaminoglykan, insbesondere die Hyaluronsäure bzw. deren Salze, bzw. Komponente (c) andererseits in einem gewichtsbezogenen Verhältnis von ([a] : [c]) im Bereich von 2 : 1 bis 500 : 1, insbesondere im Bereich von 3 : 1 bis 100 : 1, vorzugsweise im Bereich von 5 : 1 bis 50 : 1, bevorzugt im Bereich von 9 : 1 bis 30 : 1, aufweist.

Schließlich kann es erfindungsgemäß vorgesehen sein, dass die Zusammensetzung das mindestens eine Oberflächendesinfektionsmittel bzw. die Komponente (b) sowie das mindestens eine vorzugsweise saure Glykosaminoglykan, insbesondere die Hyaluronsäure bzw. deren Salze, bzw. Komponente (c) in einem gewichtsbezogenen Verhältnis von ([b] : [c]) im Bereich von 1 : 1 bis 1 : 10, insbesondere im Bereich von 1 : 2 bis 1 : 4, aufweist.

Darüber hinaus kann es erfindungsgemäß vorgesehen sein, dass die Zusammensetzung mindestens einen weiteren Wirk- und/oder Inhaltsstoff enthält. Dabei kann der weitere Wirk- und/oder Inhaltsstoff insbesondere ausgewählt sein aus der Gruppe von Schleimhautschutzmitteln, Antiseptika, Vitaminen, Spurenelementen, Mineralien, Mikronährstoffen sowie deren Mischungen.

Auch kann es im Rahmen des erfindungsgemäßen Konzepts vorgesehen sein, dass die Zusammensetzung außerdem mindestens ein Additiv enthält. Dieses Additiv kann insbesondere ausgewählt sein aus der Gruppe von Verarbeitungshilfsmitteln, Färbe-, Puffer-, Riech-, Duft-, Streck-, Binde-, Netz-und/oder Konservierungsstoffen, pH-Stellmitteln, pH-Puffersubstanzen, Verdickungsmitteln, Aromastoffen, Geschmacksstoffen, Süßstoffen und Süßungsmitteln, Säuerungsmitteln, Stabilisatoren und/oder Antiseptika sowie deren Mischungen.

Zudem hat es sich im Rahmen der vorliegenden Erfindung überraschenderweise gezeigt, dass es in Bezug auf die Linderung von Heiserkeit, Halsschmerzen oder anderen entzündlichen Erkrankungen des Mund- und Rachenraums vorteilhaft ist, wenn die Zusammensetzung zusätzlich einen Schaumbildner enthält. Ein Schaumbildner ermöglicht eine noch effizientere Benetzung der Haut bzw. Schleimhaut, so dass die Bildung des Schutzfilms auf Basis von Polysacchariden bzw. sauren Glykosaminoglykanen noch weitergehend verstärkt wird. Besonders gute Ergebnisse werden in diesem Zusammenhang mit dem Einsatz des dreiwertigen Alkohols Glycerin bzw. dessen physiologisch unbedenklichen Derivaten bzw. Estern erzielt.

Im Rahmen der vorliegenden Erfindung kann es somit vorgesehen sein, dass die Zusammensetzung als weiteren Wirk- und/oder Inhaltsstoff einen Schaumbildner, insbesondere Glycerin und/oder dessen physiologisch verträgliche Derivate oder Ester, aufweist. Was die Menge eines solchen Schaumbildners anbelangt, so kann diese gleichermaßen in weiten Bereichen variieren. Üblicherweise weist die erfindungsgemäße Zusammensetzung Glycerin bzw. dessen physiologisch verträgliche Derivate oder Ester in einer Menge im Bereich von 0,01 bis 5 Gew.-%, insbesondere im Bereich von 0,05 bis 4 Gew.-%, vorzugsweise im Bereich von 0,1 bis 2,5 Gew.-%, bevorzugt im Bereich von 0,2 bis 1 Gew.-%, bezogen auf die erfindungsgemäße Zusammensetzung, auf.

Was die Darreichungsform bzw. Applikationsform der vorliegenden Erfindung anbelangt, so ist diese äußerst vielfältig und kann gezielt auf den jeweiligen Verwendungszweck maßgeschneidert bzw. angepasst werden.

Gemäß einer ersten erfindungsgemäß bevorzugten Ausfuhrungsform kann es vorgesehen sein, dass die Zusammensetzung als feste Darreichungsform vorliegt bzw. dass die Zusammensetzung als Tablette, Dragee, Pille, Hartkaramelle oder dergleichen, insbesondere als Lutschtablette, vorliegt. In diesem Zusammenhang ist es besonders bevorzugt, wenn die Lutschtablette ein Gesamtgewicht im Bereich von 0,5 bis 5 g, insbesondere im Bereich von 0,8 bis 4 g, vorzugsweise im Bereich von 1 bis 3 g, aufweist.

Um eine gute Freisetzung der Wirk- bzw. Inhaltsstoffe zu ermöglichen, ist es bevorzugt, wenn die Zusammensetzung diese eingebettet in eine feste Matrix bzw. Masse, insbesondere eine Masse bzw. Matrix auf Basis von Zucken und/oder Zuckeraustauschstoffen, enthält. In diesem Zusammenhang kann der Zucker aus der Gruppe von Saccharose, Glukose, insbesondere Dextrose, und Fructose ausgewählt sein. Gleichermaßen kann es vorgesehen sein, dass die Zuckeraustauschstoffe aus Zuckeralkoholen, vorzugsweise aus der Gruppe von Mannit, Xylit, Sorbit, Isomalt, Maltitsirup, Lactit, Leucrose, Fructooligosacchariden, Glucanen, Polyglucose, besonders bevorzugt Isomalt, ausgewählt sind.

Was die eingesetzten Mengen an Zucker bzw. Zuckeraustauschstoffen anbelangt, so sind diese variabel. Im Rahmen der vorliegenden Erfindung ist es bevorzugt, wenn die eingesetzte Menge 30 bis 99,9 Gew.-%, insbesondere im Bereich von 40 bis 99 Gew.-%, vorzugsweise im Bereich von 50 bis 98 Gew. %, bezogen auf die Zusammensetzung, beträgt.

Was die feste Dosierungsform hinsichtlich ihrer physikalischen Ausgestaltung anbelangt, so ist es üblicherweise vorgesehen, dass die feste Dosierungsform eine therapeutisch wirksame Menge der Wirk- bzw. Inhaltsstoffe beim Lutschen freisetzt, wenn die feste Dosierungsform im Mund- und Rachenraum eines Patienten verabreicht und gelutscht wird.

Gemäß einer weiteren bevorzugten, alternativen Ausführungsform der vorliegenden Erfindung kann es vorgesehen sein, dass die Zusammensetzung als viskose bzw. pastöse Darreichungsform vorliegt. Insbesondere kann es vorgesehen sein, dass die Zusammensetzung als Salbe, Creme, Paste, Gel oder dergleichen vorliegt.

In diesem Zusammenhang ist es üblicherweise vorgesehen, dass die Zusammensetzung wässrig oder wässrig-alkoholisch oder organisch basiert ist.

Darüber hinaus kann es hierbei erfindungsgemäß vorgesehen sein, dass die Zusammensetzung Wasser enthält. Insbesondere liegt in der erfindungsgemäßen Zusammensetzung Wasser in einer relativen Menge von mindestens 10 Gew.-%, insbesondere mindestens 20 Gew.-%, vorzugsweise mindestens 30 Gew.-%, bezogen auf die Zusammensetzung, vor.

Darüber hinaus kann es im Hinblick auf viskose bzw. pastöse Darreichungsformen vorgesehen sein, dass die Zusammensetzung mindestens einen Trägerstoff aufweist. In diesem Zusammenhang ist es besonders bevorzugt, wenn der Trägerstoff aus der Gruppe von Lösungsmitteln, Lösungsvermittlern, Emulgatoren und dergleichen ausgewählt ist. Besonders bevorzugt ist der Trägerstoff aus der Gruppe von Ethanol, Isopropanol, Ethylcarbonat, Ethylacetat, Benzylalkohol, Benzylbenzoat, Propylenglycol, 1,3-Butylglycol sowie deren Kombinationen ausgewählt.

Sofern im Rahmen der vorliegenden Erfindung die Bereitstellung einer organisch basierten Zusammensetzung vorgesehen ist, so enthält diese vorzugsweise ebenfalls mindestens einen Trägerstoff, wobei in diesem Fall der Trägerstoff vorzugsweise ein Öl bzw. Fett ist. Insbesondere kann der Trägerstoff ausgewählt sein aus Wollfett, Baumwollsaatöl, Vaseline, Glycerinfettsäureestern, Polyethylenglykolen sowie deren Mischungen und Kombinationen.

Um eine gute Applikation einerseits und eine gute Haftung der Zusammensetzung an der Haut bzw. Schleimhaut, insbesondere im Mund- bzw. Rachenraum, zu ermöglichen, ist es vorteilhaft, die erfindungsgemäße Zusammensetzung in viskoser bzw. pastöser Form auf eine definierte Viskosität einzustellen. Dabei sollte die Zusammensetzung weder - im Hinblick auf die Haftung - zu dünnflüssig sein, noch übermäßig dickflüssig bzw. zäh sein, insbesondere im Hinblick auf die Applizierbarkeit. Als besonders bevorzugt hat sich erwiesen, wenn die erfindungsgemäße Zusammensetzung eine Brookfield-Viskosität bei einer Temperatur von 25 °C im Bereich von 500 bis 100.000 mPas, insbesondere im Bereich von 750 bis 50.000 mPas, bevorzugt im Bereich von 1.000 bis 10.000 mPas, besonders bevorzugt im Bereich von 1.500 bis 8.000 mPas, ganz besonders bevorzugt im Bereich von 2.000 bis 6.000 mPas, aufweist.

Darüber hinaus kann es - insbesondere im Fall von wässrigen bzw. wässrig-alkoholisch basierten Zusammensetzungen - vorgesehen sein, dass die Zusammensetzung mindestens einen Gelbildner aufweist.

Vorzugsweise kann der Gelbildner aus der Gruppe von Bentoniten, Kieselsäuren, Polyacrylsäuren, Carbomeren, Polyvinylpyrrolidonen, Cellulose und Cellulosederivaten, Xanthanen sowie deren Mischungen, vorzugsweise Cellulose und Cellulosederivaten, ausgewählt sein. Insbesondere kann der Gelbildner aus modifizierten Cellulosen, insbesondere chemisch modifizierten Cellulosen, besonders bevorzugt Methylcellulose, Hydroxymethylcellulose, Carboxymethylcellulose und/oder Hydroxyethylcellulose, ausgewählt sein. Besonders bevorzugt enthält die Zusammensetzung als Gelbildner Hydroxymethylcellulose.

Was die eingesetzte Menge des Gelbildners anbelangt, so ist diese variabel. Vorzugsweise enthält die Zusammensetzung nach der Erfindung den Gelbildner in einer Menge im Bereich von 0,01 bis 20 Gew.-%, insbesondere im Bereich 0,1 bis 10 Gew.-%, vorzugsweise im Bereich 0,5 bis 5 Gew.-%, bevorzugt 1 bis 3 Gew.-%, bezogen auf die Zusammensetzung.

Gemäß einer weiteren bevorzugten, alternativen Ausführungsform der vorliegenden Erfindung kann es vorgesehen sein, dass die erfindungsgemäße Zusammensetzung als flüssige Darreichungsform vorliegt. Dabei kann es sich insbesondere um ein Fluid, Mundwasser, eine Mundspülung, Saft, Gurgellösung der dergleichen handeln.

In Bezug auf die Bereitstellung der erfindungsgemäßen Zusammensetzung als flüssige Darreichungsform ist es üblicherweise vorgesehen, dass die Zusammensetzung wässrig oder wässrig-alkoholisch basiert ist.

In diesem Zusammenhang ist es insbesondere bevorzugt, wenn die Zusammensetzung Wasser enthält. Insbesondere liegt in der erfindungsgemäßen Zusammensetzung Wasser in einer Menge von mindestens 10 Gew.-%, insbesondere mindestens 20 Gew.-%, vorzugsweise mindestens 30 Gew.-%, bezogen auf die Zusammensetzung, und/oder insbesondere in einer Menge im Bereich von 10 bis 98 Gew.-%, insbesondere im Bereich 20 bis 95 Gew.-%, vorzugsweise 30 bis 90 Gew.-%, bezogen auf die Zusammensetzung, vor.

Um in der erfindungsgemäßen Zusammensetzung bzw. insbesondere bei deren Applikation einen konstanten pH-Wert, insbesondere einen physiologisch verträglichen pH-Wert, zu ermöglichen, ist es bevorzugt, wenn die Zusammensetzung mindestens eine Säure und/oder Base, insbesondere zur Ausbildung eines Puffersystems, enthält. In diesem Zusammenhang kann es gleichermaßen vorgesehen sein, dass die Zusammensetzung mindestens ein Puffersystem enthält bzw. mindestens eine Base aufweist. Insbesondere kann es in diesem Zusammenhang vorgesehen sein, dass die Base ausgewählt ist aus der Gruppe von Aminen, Carboxylaten, Alkali- und/oder Erdalkalimetallhydroxiden sowie deren Mischungen und Kombinationen, insbesondere Alkali- und Erdalkalimetallhydroxiden, bevorzugt Natriumhydroxid.

Im Rahmen dieser Ausführungsform kann es zudem vorgesehen sein, dass die flüssige Zusammensetzung als Spray, Aerosol oder dergleichen vorliegt. In diesem Zusammenhang ist es üblicherweise vorgesehen, dass die Zusammensetzung in dem Fachmann wohlbekannten Sprüh- bzw. Pumpvorrichtungen vorliegt, so dass es diesbezüglich keiner weiteren Ausführungen bedarf.

Gegenstand der vorliegenden Erfindung ist - gemäß einer besonderen Ausführungsform - somit eine Zusammensetzung, insbesondere pharmazeutische Zusammensetzung, insbesondere wie sie zuvor beschrieben wurde, vorzugsweise in Form einer festen oder flüssigen, bevorzugt festen Dosierung, welche insbesondere zur topischen Behandlung von Heiserkeit bzw. entzündlichen Erkrankungen des Mund- und Rachenraums geeignet ist, wobei die Zusammensetzung - in Kombination und jeweils in wirksamen, insbesondere pharmazeutisch wirksamen, Mengen -
(a) mindestens ein virustatisches oder antiviral wirksames Polysaccharid, ausgewählt aus Carrageenen und/oder deren physiologisch verträglichen Salzen, Galactansulfaten und/oder deren physiologisch verträglichen Salzen und Polyuroniden und/oder deren physiologisch verträglichen Salzen, in einer Menge im Bereich von 0,1 bis 40 Gew.-%, insbesondere im Bereich 0,5 bis 30 Gew.-%, vorzugsweise im Bereich von 1 bis 25 Gew.-%, bevorzugt im Bereich von 2 bis 20 Gew.-%, besonders bevorzugt im Bereich von 3 bis 18 Gew.-%, bezogen auf die Zusammensetzung;
(b) Polyhexanid als Oberflächendesinfektionsmittel in einer Menge im Bereich von 0,001 bis 5 Gew.-%, insbesondere im Bereich von 0,002 bis 2,5 Gew.-%, vorzugsweise im Bereich von 0,005 bis 2 Gew.-%, bevorzugt im Bereich von 0,01 bis 1 Gew.-%, besonders bevorzugt im Bereich von 0,05 bis 0,5 Gew.-%, bezogen auf die Zusammensetzung; sowie
(c) Hyaluronsäure oder deren Salze in einer Menge im Bereich von 0,01 bis 10 Gew.-%, insbesondere im Bereich von 0,05 bis 5 Gew.-%, vorzugsweise im Bereich von 0,1 bis 2 Gew.-%, bezogen auf die Zusammensetzung;
(d) gegebenenfalls mindestens ein Lokalanästhetikum in einer Menge im Bereich von 0,01 bis 5 Gew.-%, insbesondere im Bereich von 0,05 bis 2,5 Gew.-%, vorzugsweise im Bereich von 0,01 bis 1,5 Gew.-%, bevorzugt im Bereich von 0,1 bis 1 Gew.-%, bezogen auf die Zusammensetzung;
aufweist.

Was die Verwendung der erfindungsgemäßen Zusammensetzung anbelangt, so ist diese insbesondere zur Verwendung im Bereich der Medizin, Pharmazie oder Lebensmitteltechnik geeignet. Insbesondere ist die erfindungsgemäße Zusammensetzung zur Verwendung im Bereich der Humanmedizin oder Veterinärmedizin geeignet.

Die Zusammensetzung nach der vorliegenden Erfindung eignet sich insbesondere zur Verwendung bei der prophylaktischen bzw. therapeutischen Behandlung von Heiserkeit bzw. entzündlichen Erkrankungen des Mund- und Rachenraums, insbesondere Halsschmerzen, insbesondere zur topischen Behandlung von Heiserkeit oder Halsschmerzen.

Darüber hinaus ist die pharmazeutische Zusammensetzung, wie sie zuvor definiert wurde, zur Herstellung eines Medikaments oder Therapeutikums zur phrophylaktischen bzw. therapeutischen Behandlung von Heiserkeit bzw. entzündlichen Erkrankungen des Mund- und Rachenraums, insbesondere Halsschmerzen, insbesondere zur topischen Behandlung von Heiserkeit oder Halsschmerzen, geeignet.

Für weitergehende Einzelheiten zu der erfindungsgemäßen Verwendung kann - zur Vermeidung unnötiger Wiederholungen - auf obige Ausführungen zu der erfindungsgemäßen Zusammensetzung verwiesen werden, welche im Bezug auf die erfindungsgemäße Verwendung entsprechend gelten.

Weitere Ausgestaltungen, Abwandlungen und Variationen sowie Vorteile der vorliegenden Erfindung sind für den Fachmann beim Lesen der Beschreibung ohne Weiteres erkennbar und realisierbar, ohne dass er dabei den Rahmen der vorliegenden Erfindung verlässt.

Die vorliegende Erfindung wird anhand der folgenden Ausführungsbeispiele veranschaulicht, welche die vorliegende Erfindung jedoch keinesfalls beschränken.

### Ausführungsbeispiele :

### A) Herstellung erfindungsgemäßer Zusammensetzungen in Form von Lutschtabletten auf Hartkaramellenbasis und von Vergleichszusammensetzungen gleichermaßen in Form von Lutschtabletten auf Hartkaramellenbasis

Es wurden verschiedene erfindungsgemäße und nicht erfindungsgemäße Zusammensetzungen in Form von Lutschtabletten auf Hartkaramellenbasis hergestellt, wobei als Zuckeraustauschstoff zur Einlagerung bzw. Einbettung der Wirk- und Inhaltsstoffe jeweils Xylitol und Mannitol zu gleichen Gewichtsteilen eingesetzt wurden. Gleichermaßen können alternativ aber auch Fructose- und/oder Glucosesirup bzw. andere Süßstoffe oder Zuckeraustauschstoffe als Matrixmaterial verwendet werden. Die Herstellung der Lutschtabletten bzw. Hartkaramellen erfolgte in dem Fachmann an sich bekannter Weise.

Die Ausgangsstoffe (Wirkstoffe, Geschmacksstoffe, Zusatz- bzw. Hilfsstoffe etc.) wurden hierzu entsprechend der jeweiligen Rezeptur eingewogen und einzeln in das Matrixmaterial (Xylitol und Mannitol) eingemischt. Die Zugabe von Wasser war nicht notwendig. Der Vorgang fand unter Erwärmung statt. Die homogene Mischung aller Rohstoffe wurde in eine Dosiermaschine oder Dosiereinheit überführt. Dort wurden die Lutschtabletten in ihrer entsprechenden Größe hergestellt und während des Prozesses gewogen. Es resultierten Trockengewichte von 2,5 g pro Applikationseinheit bzw. pro Lutschtablette. Auf diese Weise wurden sowohl erfindungsgemäße Hartkaramellen als auch nichterfindungsgemäße Hartkaramellen zu Vergleichszwecken hergestellt.

Die erfindungsgemäßen Hartkaramellen A, B und C enthielten als Wirkstoffe jeweils 10 Gew.-% Carrageen als Polysaccharid sowie 0,25 Gew.-% Polyhexanid als Oberflächendesinfektionsmittel. Die Hartkaramellen B und C enthielten darüber hinaus jeweils 0,2 Gew.-% Natriumhyaluronat als saures Glykosaminoglykan. Hartkaramelle C enthielt zudem als vierte Wirkstoff-komponente 0,5 Gew.-% Polidocanol als Lokalanästhetikum. Die prozentualen Gewichtsanteile sind jeweils auf das Trockengewicht der Zusammensetzung bezogen. Die vollständigen Rezepturen der erfindungsgemäßen Hartkaramellen A, B und C sind der nachstehenden Tabelle 1 zu entnehmen.

**Tabelle 1: Rezepturen der erfindungsgemäßen Hartkaramellen A, B und C**

| **Inhaltsstoff** | **Menge (trocken) pro Lutschtablette A [Gew.-%]** | **Menge (trocken) pro Lutschtablette B [Gew.-%]** | **Menge (trocken) pro Lutschtablette C [Gew.-%]** |
|---|---|---|---|
| Glycerin | 0,25 | 0,25 | 0,25 |
| Carrageen | 10 | 10 | 10 |
| Polyhexanid | 0,25 | 0,25 | 0,25 |
| Natriumhyaluronat | - | 0,2 | 0,2 |
| Polidocanol | - | - | 0,5 |
| Matrixbildner | 77,41 | 77,21 | 76,71 |
| Hilfsstoffe | 11,75 | 11,75 | 11,75 |
| Geschmacksbildner | 0,34 | 0,34 | 0,34 |

Die nichterfindungsgemäßen Vergleichstabletten D und E unterschieden sich im Bezug auf die Wirkstoffe von den erfindungsgemäßen Vergleichstabletten dahingehend, dass sie nicht die erfindungsgemäße Wirkstoffkombination enthielten, sondern vielmehr Monopräparate der erfindungsgemäß eingesetzten Wirkstoffe darstellten: Die Vergleichstablette D enthielt als einzigen Wirkstoff das Polysaccharid Carrageen in einer Menge von 10 Gew.-%, bezogen auf das Trockengewicht der Zusammensetzung. Die Vergleichstabelle E enthielt als einzigen Wirkstoff 0,25 Gew.-% Polyhexanid, bezogen auf das Trockengewicht der Zusammensetzung. Die gegenüber den erfindungsgemäßen Hartkaramellen fehlenden Inhaltsstoffe wurden durch äquivalente gewichtsbezogene Mengen an Matrixmaterial ersetzt.

### B) Wirksamkeits- und Anwendungsstudien mit den erfindungsgemäßen Lutschtabletten A, B und C sowie den Vergleichslutschtabletten D und E und Therapiemaßnahmen des Standes der Technik

Als erfindungsgemäße Lutschtabletten sowie die Vergleichslutschtabletten wurden die zuvor unter A) beschriebenen Lutschtabletten A bis E eingesetzt. Darüber hinaus wurden im Rahmen der Anwendungs- und Wirksamkeitsstudien Gurgellösungen auf Basis von Salbei und Salz sowie handelsübliche Hustenbonbons zu Vergleichszwecken im Rahmen der Wirksamkeits- und Anwendungsstudien eingesetzt.

Zur Untersuchung der Wirksamkeit der erfindungsgemäßen Lutschtabletten A bis C, der Vergleichslutschtabletten D und E und den Therapiemaßnahmen des Standes der Technik (d. h. Gurgellösungen bzw. Hustenbonbons) wurde eine Probandengruppe mit insgesamt 105 Probanden im Alter von 18 bis 75 Jahren, von denen 67 männlich und 38 weiblich waren, herangezogen. Die Probanden litten an Heiserkeit sowie entzündlichen Erkrankungen des Hals-und Rachenraums aufgrund von grippalen Infekten und der damit einhergehenden Schmerz- und Entzündungssymptomatik.

Jeweils 15 Probanden erhielten dreimal täglich über einen Zeitraum von 3 Tagen jeweils eine der Lutschtabletten A bis E. Die Probandengruppe F erhielt über einen Zeitraum von 3 Tagen ebenfalls dreimal täglich eine Gurgellösung auf Basis von Salzwasser und Salbei. Darüber hinaus erhielt die Probandengruppe G dreimal täglich ein handelsübliches Hustenbonbon verabreicht. Im Rahmen der Behandlung wurde die jeweils getestete Zusammensetzung bzw. Therapiemaßnahme in Bezug auf den Rückgang der Beeinträchtigung der Phonation, die Linderung der Schmerzsymptomatik, die Nachhaltigkeit der Behandlung, insbesondere in Bezug auf die Schmerzsymptomatik, eine Linderung der Entzündungssymptomatik sowie das Mundgefühl nach dem Schulnotensystem (1 = sehr gut bis 6 = ungenügend) bewertet. Die diesbezüglichen Ergebnisse sind der nachstehenden Tabelle 2 zu entnehmen.

**Tabelle 2: Bewertung der erfindungsgemäßen Zusammensetzungen sowie der Vergleichszusammensetzungen bzw. der Therapiemaßnahmen des Standes der Technik**

| **Zusammensetzung** | **Rückgang der Beeinträchtigung der Phonation** | **Linderung der Schmerzsymptomatik** | **Nachhaltigkeit der Behandlung (Schmerzsymp-tomatik)** | **Linderung der Entzündungssymptomatik** | **Mundgefühl** |
|---|---|---|---|---|---|
| A (erfindungs-gemäß) | 1,7 | 1,8 | 1,9 | 1,7 | 1,7 |
| B (erfindungsgemäß) | 1,5 | 1,7 | 1,8 | 1,6 | 1,6 |
| C (erfindungs-gemäß) | 1,4 | 1,2 | 1,1 | 1,4 | 1,6 |
| D (Vergleich) | 2,6 | 2,7 | 3,1 | 3,0 | 2,0 |
| E (Vergleich) | 3,6 | 3,1 | 3,9 | 2,6 | 1,8 |
| F (Stand der Technik) | 4,1 | 4,5 | 4,6 | 5,0 | 4,5 |
| G (Stand der Technik) | 4,5 | 4,1 | 4,7 | 5,1 | 2,7 |

Mit allen drei erfindungsgemäßen Lutschtabletten wurden in Bezug auf sämtliche untersuchte Bewertungskriterien hervorragende Ergebnisse im sehr guten Bereich erzielt. Die besten Ergebnisse wurden mit der erfindungsgemäßen Lutschtablette C erzielt. Insbesondere der Einsatz des Lokalanästhetikums Polidocanol führt zu einer noch weitergehenden Linderung der Schmerzsymptomatik, und dies auch im Hinblick auf die Nachhaltigkeit der Schmerzlinderung. Mit der erfindungsgemäßen Lutschtablette B, welche zusätzlich das Glykosaminoglykan Hyaluronsäure enthielt, wurden insbesondere in Bezug auf den Rückgang der Beeinträchtigung der Phonation noch bessere Ergebnisse als mit der erfindungsgemäßen Lutschtablette A erzielt. Der zusätzliche Einsatz von Hyaluronsäure führt - ohne sich hierbei auf diese Theorie beschränken zu wollen - vermutlich zu einer noch weitergehend verbesserten Benetzung der Schleimhaut, so dass insgesamt noch besser einer Austrocknung vorgebeugt werden kann. Wie die vorstehenden Ausführungen zeigen, eignen sich jedoch - trotz der leichten Unterschiede hinsichtlich der Wirkeffizienz - alle erfindungsgemäßen Lutschtabletten gleichermaßen zur Behandlung von Heiserkeit bzw. entzündlichen Erkrankungen des Mund- und Rachenraums, insbesondere Halsschmerzen.

Mit den Vergleichszusammensetzungen D und E hingegen konnten keine zufriedenstellenden Ergebnisse erzielt werden. Insbesondere die Schmerz- und Entzündungssymptomatik konnte mit den Vergleichszusammensetzungen D und E nur äußerst unzureichend gelindert werden. Die Monopräparate der eingesetzten Wirkstoffe sind somit bei alleiniger Applikation nicht zur effektiven Behandlung von Heiserkeit bzw. entzündlichen Erkrankungen des Mund- und Rachenraums geeignet. Vielmehr lassen die starken Differenzen in Bezug auf die Wirkeffizienz der jeweiligen Einzelwirkstoffe im Vergleich zur Wirkung bei gemeinsamer Verabreichung auf eine gegenseitige Wirkverstärkung schließen, was als Indiz für das Vorliegen eines synergistischen Effekts zu werten ist.

Die schlechtesten Ergebnisse wurden insgesamt mit den beiden Behandlungsmethoden des Standes der Technik erzielt. Weder mit Gurgellösungen F noch mit herkömmlichen Hustenbonbons B lassen sich die mit Heiserkeit und entzündlichen Erkrankungen des Mund- und Rachenraums einhergehenden Beschwerden bzw. Symptome in effizienter Weise lindern. Insbesondere bewirken derartige Therapieformen weder eine Linderung der Schmerzsymptomatik noch der Entzündungssymptomatik. Auch ein beschleunigter Rückgang der Beeinträchtigung der Phonation konnte mit diesen beiden Zusammensetzungen nicht erzielt werden.

Die Untersuchungen zeigen insgesamt, dass die Anwendung der erfindungsgemäßen Zusammensetzungen bei Heiserkeit bzw. entzündlichen Erkrankungen des Hals- und Rachenraums sämtliche im Allgemeinen damit einhergehenden Beschwerden lindert; insbesondere tritt eine Linderung der Schmerz- und Entzündungssymptomatik ein. Darüber hinaus führt die Applikation der erfindungsgemäßen Zusammensetzungen zu einem beschleunigten Rückgang der Beeinträchtigung der Phonation. Auch ist das Mundgefühl der erfindungsgemäßen Zusammensetzung gegenüber den aus dem Stand der Technik Behandlungsmethoden verbessert. Ohne sich hierbei auf eine Theorie beschränken zu wollen, ist dies möglicherweise auf die benetzende Wirkung des eingesetzten Polysaccharids einerseits sowie der Hyaluronsäure andererseits und gegebenenfalls der Hyaluronsäure zurückzuführen.

## Patentansprüche

1. Zusammensetzung, insbesondere pharmazeutische Zusammensetzung, vorzugsweise in Form einer festen oder flüssigen, insbesondere festen Dosierung, insbesondere zur topischen Behandlung von Heiserkeit oder Halsschmerzen und/oder entzündlichen Erkrankungen des Mund- und Rachenraums,
wobei die Zusammensetzung - in Kombination und jeweils in wirksamen, insbesondere pharmazeutisch wirksamen Mengen -
(a) mindestens ein virustatisches und/oder antiviral wirksames Polysaccharid, ausgewählt aus Carrageenen und/oder deren physiologisch verträglichen Salzen, Galactansulfaten und/oder deren physiologisch verträglichen Salzen und Polyuroniden und/oder deren physiologisch verträglichen Salzen ("Komponente (a)");
(b) Polyhexanid als Oberflächendesinfektionsmittel, ("Komponente (b)"); sowie
(c) Hyaluronsäure oder deren Salze ("Komponente (c)");
aufweist.

2. Zusammensetzung nach Anspruch 1,
wobei die Komponente (a) ein gewichtsmittleres Molekulargewicht im Bereich von 5.000 bis 10.000.000 Da, insbesondere im Bereich von 15.000 bis 7.500.000 Da, vorzugsweise im Bereich von 20.000 bis 5.000.000 Da, bevorzugt im Bereich von 30.000 bis 3.000.000 Da, aufweist, insbesondere bestimmt mittels Gelpermeationschromatographie (GPC), vorzugsweise nach DIN 55672; und/oder
wobei die Komponente (a) ein Biopolymer und/oder ein Polymer biogenen Ursprungs ist; und/oder
wobei die Komponente (a) ausgewählt ist aus Homopolysacchariden und/oder Heteropolysacchariden, vorzugsweise Heteropolysacchariden.

3. Zusammensetzung nach einem der vorangehenden Ansprüche,
wobei die Komponente (a), insbesondere das Polysaccharid, vorzugsweise als Schleimstoff, bevorzugt in Form einer Droge, insbesondere in Form zerkleinerter oder pulverisierter oder extrahierter Algen, Pflanzenteile oder Pflanzenbestandteile, bereitgestellt ist; und/oder
wobei die Komponente (a), insbesondere das Polysaccharid, insbesondere als Schleimstoff, vorzugsweise in Form eines Extrakts, insbesondere in Form eines Trockenextrakts, bereitgestellt ist, insbesondere wobei der Extrakt ausgehend von einem wässrigen, alkoholischen oder wässrig-alkoholischen Extrakt, bevorzugt einem wässrigen Extrakt, erhältlich ist; insbesondere wobei der Extrakt, vorzugsweise der Trockenextrakt, ein Droge/Extrakt-Verhältnis (DEV), insbesondere berechnet als gewichtsbezogenes Verhältnis von eingesetzter Ausgangsmenge an Droge zu erhaltenem Extrakt, im Bereich von 0,1 : 1 bis 50 : 1, insbesondere im Bereich von 0,5 : 1 bis 25 : 1, vorzugsweise im Bereich von 2 : 1 bis 20 : 1, besonders bevorzugt im Bereich von 5 : 1 bis 10 : 1, aufweist.

4. Zusammensetzung nach einem der vorangehenden Ansprüche,
wobei die Komponente (a), insbesondere das Polysaccharid, vorzugsweise der Schleimstoff, erhältlich ist aus Isländischem Moos *(Lichen islandicus),* Eibisch *(Althaea officinalis L*.), Spitzwegerich *(Plantago lanceolata L.),* Malve *(Malva sylvestris L.* und *M. neglecta WALLR.),* Boxhornklee (*Trigonella foenum-graecum L.),* Salep, Quitte (*Cydonia oblonga MILL.)* und Algen, vorzugsweise Rotalgen, insbesondere Rotalgen-Spezies der Gattungen *Chondrus, Euchema* und/oder *Gigartina,* sowie deren Kombinationen; und/oder
wobei die Komponente (a) ausgewählt ist aus den alpha-, iota-, gamma-, kappa-und/oder lambda-Carrageenen und/oder deren Derivaten und/oder deren physiologisch verträglichen Salzen, vorzugsweise iota-Carrageen und/oder dessen Derivaten und/oder physiologisch verträglichen Salzen, und/oder wobei die Komponente (a), insbesondere das Polysaccharid, vorzugsweise der Schleimstoff, ein Heteropolymer auf Basis von alpha-, iota-, gamma-, kappa- und/oder lambda-Carrageenen und/oder deren Derivaten und/oder deren physiologisch verträglichen Salzen, insbesondere auf Basis von iota-und kappa-Carrageen und/oder deren Derivaten und/oder deren physiologisch verträglichen Salzen, ist; und/oder
wobei die Komponente (a), insbesondere das Polysaccharid, vorzugsweise der Schleimstoff, erhältlich ist durch Extraktion von Algen, vorzugsweise Rotalgen, besonders bevorzugt Rotalgen-Spezies der Gattungen *Chondrus, Euchema* und/oder *Gigartina.*

5. Zusammensetzung nach einem der vorangehenden Ansprüche,
wobei die Zusammensetzung die Komponente (a) in einer relativen Menge im Bereich von 0,1 bis 40 Gew.-%, insbesondere im Bereich von 0,5 bis 30 Gew.-%, vorzugsweise im Bereich von 1 bis 25 Gew.-%, bevorzugt im Bereich von 2 bis 20 Gew.-%, besonders bevorzugt im Bereich von 3 bis 18 Gew.-%, bezogen auf die Zusammensetzung, enthält; und/oder
wobei die Zusammensetzung die Komponente (a) in einer absoluten Menge im Bereich von 0,001 bis 5 g, insbesondere im Bereich von 0,005 bis 4 g, vorzugsweise im Bereich von 0,01 bis 3 g, bevorzugt im Bereich von 0,05 bis 2 g, insbesondere bezogen auf eine Applikationseinheit und/oder insbesondere auf eine Applikationsmenge, enthält; und/oder
wobei die Zusammensetzung die Komponente (b) in einer Menge im Bereich von 0,001 bis 5 Gew.-%, insbesondere im Bereich von 0,01 bis 2,5 Gew.-%, vorzugsweise im Bereich von 0,05 bis 2 Gew.-%, bevorzugt im Bereich von 0,01 bis 1 Gew.-%, besonders bevorzugt im Bereich von 0,05 bis 0,5 Gew.-%, bezogen auf die Zusammensetzung, enthält.

6. Zusammensetzung nach einem der vorangehenden Ansprüche,
wobei die Komponente (c) das Natriumsalz der Hyaluronsäure ist; und/oder
wobei die Zusammensetzung die Komponente (c) in einer Menge im Bereich von 0,01 bis 10 Gew.-%, insbesondere im Bereich von 0,05 bis 5 Gew.-%, vorzugsweise im Bereich von 0,1 bis 1 Gew.-%, bezogen auf die Zusammensetzung, enthält; und/oder
wobei die Zusammensetzung die Komponente (d) in einer relativen Menge im Bereich von 0,01 bis 5 Gew.-%, insbesondere im Bereich von 0,05 bis 2,5 Gew.-%, vorzugsweise im Bereich von 0,01 bis 1,5 Gew.-%, bevorzugt im Bereich von 0,1 bis 1 Gew.-%, bezogen auf die pharmazeutische Zusammensetzung, enthält; und/oder
wobei die Zusammensetzung als weitere Komponente (d) mindestens ein Lokalanästhetikum aufweist; und/oder
insbesondere wobei die Komponente (d) ausgewählt ist aus der Gruppe von Polidocanol, Benzocain, Lidocain und/oder Ambroxol, insbesondere wobei die Komponente (d) Polidocanol ist; und/oder
insbesondere wobei die Zusammensetzung die Komponente (d) in einer relativen Menge im Bereich von 0,01 bis 5 Gew.-%, insbesondere im Bereich von 0,05 bis 2,5 Gew.-%, vorzugsweise im Bereich von 0,01 bis 1,5 Gew.-%, bevorzugt im Bereich von 0,1 bis 1 Gew.-%, bezogen auf die pharmazeutische Zusammensetzung, enthält.

7. Zusammensetzung nach einem der vorangehenden Ansprüche,
wobei die Zusammensetzung die Komponente (d) in einer absoluten Menge im Bereich von 0,01 bis 500 mg, insbesondere im Bereich von 0,05 bis 250 mg, vorzugsweise im Bereich von 0,1 bis 150 mg, bevorzugt im Bereich von 0,5 bis 50 mg, besonders bevorzugt im Bereich von 1 bis 20 mg, bezogen auf eine Applikationseinheit, enthält; und/oder
wobei die Zusammensetzung die Komponente (a) und die Komponente (b) in einem gewichtsbezogenen Verhältnis von ([a] : [b]) im Bereich von 1 : 1 bis 1.000 : 1, insbesondere im Bereich von 5 : 1 bis 500 : 1, vorzugsweise im Bereich von 15 : 1 bis 100 : 1, bevorzugt im Bereich von 20 : 1 bis 70 : 1, aufweist und/oder
wobei die Zusammensetzung die Komponente (a) und die Komponente (c) in einem gewichtsbezogenen Verhältnis von ([a] : [c]) im Bereich von 2 : 1 bis 500 : 1, insbesondere im Bereich von 3 : 1 bis 100 : 1, vorzugsweise im Bereich von 5 : 1 bis 50 : 1, bevorzugt im Bereich von 9 : 1 bis 30 : 1, aufweist und/oder
wobei die Zusammensetzung die Komponente (b) und die Komponente (c) in einem gewichtsbezogenen Verhältnis von ([b] : [c]) im Bereich von 1 : 1 bis 1 : 10, insbesondere im Bereich von 1 : 2 bis 1 : 4, aufweist.

8. Zusammensetzung nach einem der vorangehenden Ansprüche,
wobei die Zusammensetzung mindestens einen weiteren Wirk- und/oder Inhaltsstoff, insbesondere ausgewählt aus der Gruppe von Schleimhautschutzmitteln, Antiseptika, Vitaminen, Spurenelementen, Mineralien, Mikronährstoffen sowie deren Mischungen, enthält; und/oder
wobei die Zusammensetzung außerdem mindestens ein Additiv, insbesondere ausgewählt aus der Gruppe von Verarbeitungshilfsmitteln, Färbe-, Puffer-, Riech-, Duft-, Streck-, Binde-, Netz- und/oder Konservierungsstoffen, pH-Stellmitteln, pH-Puffersubstanzen, Verdickungsmitteln, Aromastoffen, Geschmacksstoffen, Süßstoffen und Süßungsmitteln, Säuerungsmitteln, Stabilisatoren und/oder Antiseptika sowie deren Mischungen, enthält; insbesondere wobei die Zusammensetzung als Additiv Glycerin und/oder dessen physiologisch verträgliche Derivate oder Ester aufweist, insbesondere in einer Menge im Bereich von 0,01 bis 5 Gew.-%, insbesondere im Bereich von 0,05 bis 4 Gew.-%, vorzugsweise im Bereich von 0,1 bis 2,5 Gew.-%, bevorzugt im Bereich von 0,2 bis 1 Gew.-%, bezogen auf die pharmazeutische Zusammensetzung.

9. Zusammensetzung nach einem der vorangehenden Ansprüche, wobei die Zusammensetzung als feste Darreichungsform vorliegt und/oder wobei die Zusammensetzung als Tablette, Dragee, Pille, Hartkaramelle oder dergleichen, insbesondere als Lutschtablette, vorliegt, insbesondere wobei die Lutschtablette ein Gesamtgewicht im Bereich von 0,5 bis 5 g, insbesondere im Bereich von 0,8 bis 4 g, vorzugsweise im Bereich von 1 bis 3 g, aufweist;
insbesondere wobei die Zusammensetzung die Wirk- und/oder Inhaltsstoffe in einer festen Matrix und/oder Masse, insbesondere in einer Matrix und/oder Masse auf Basis von Zuckern und/oder Zuckeraustauschstoffen aufweist, insbesondere wobei die Zucker ausgewählt sind aus der Gruppe von Saccharose, Glucose, insbesondere Dextrose, und Fructose und/oder insbesondere wobei die Zuckeraustauschstoffe ausgewählt sind aus Zuckeralkoholen, vorzugsweise aus der Gruppe von Mannit, Xylit, Sorbit, Isomalt, Maltitsirup, Lactit, Leucrose, Fructooligosacchariden, Glucanen, Polyglucose, besonders bevorzugt Isomalt; und/oder
insbesondere wobei die Zusammensetzung die Zucker und/oder die Zuckeraustauschstoffe in einer relativen Menge im Bereich von 30 bis 99,9 Gew.-%, insbesondere im Bereich von 40 bis 99 Gew.-%, vorzugsweise im Bereich von 50 bis 98 Gew.-%, bezogen auf die Zusammensetzung, enthält; und/oder
insbesondere wobei die feste Dosierungsform, insbesondere die Lutschtablette, derart gebildet ist, dass die feste Dosierungsform, insbesondere die Lutschtablette, eine therapeutisch wirksame Menge der Wirk- und/oder Inhaltsstoffe beim Lutschen freisetzt, wenn die feste Dosierungsform im Mund- und Rachenraum eines Patienten verabreicht und gelutscht wird.

10. Zusammensetzung nach einem der Ansprüche 1 bis 8, wobei die Zusammensetzung als viskose und/oder pastöse Darreichungsform vorliegt und/oder wobei die Zusammensetzung als Salbe, Creme, Paste, Gel oder dergleichen vorliegt;
insbesondere wobei die Zusammensetzung wässrig, alkoholisch oder wässrigalkoholisch oder organisch basiert ist; und/oder
insbesondere wobei die Zusammensetzung Wasser enthält, insbesondere in einer relativen Menge von mindestens 10 Gew.-%, insbesondere mindestens 20 Gew.-%, vorzugsweise mindestens 30 Gew.-%, bezogen auf die Zusammensetzung, und/oder insbesondere in einer relativen Menge im Bereich von 10 bis 90 Gew.-%, insbesondere im Bereich von 20 bis 85 Gew.-%, vorzugsweise im Bereich von 30 bis 80 Gew.-%, bezogen auf die Zusammensetzung; und/oder
insbesondere wobei die Zusammensetzung mindestens einen Trägerstoff enthält, insbesondere wobei der Trägerstoff ausgewählt ist aus der Gruppe von Lösungsmitteln, Lösungsvermittlern, Emulgatoren und dergleichen, und/oder wobei der Trägerstoff ausgewählt ist aus der Gruppe von Ethanol, Isopropanol, Ethylcarbonat, Ethylacetat, Benzylalkohol, Benzylbenzoat, Propylenglycol, 1,3-Butylglycol sowie deren Kombinationen.

11. Zusammensetzung nach Anspruch 10,
wobei die Zusammensetzung mindestens einen Trägerstoff enthält, insbesondere wobei der Trägerstoff ein Öl und/oder Fett ist und/oder insbesondere wobei der Trägerstoff ausgewählt ist aus Wollfett, Baumwollsaatöl, Vaseline, Glycerinfettsäureestern, Polyethylenglykolen sowie deren Kombinationen;
insbesondere wobei die Zusammensetzung bei einer Temperatur von 25 °C eine Brookfield-Viskosität im Bereich von 500 bis 100.000 mPas, insbesondere im Bereich von 750 bis 50.000 mPas, bevorzugt im Bereich von 1.000 bis 10.000 mPas, besonders bevorzugt im Bereich von 1.500 bis 8.000 mPas, ganz besonders bevorzugt im Bereich von 2.000 bis 6.000 mPas, aufweist; und/oder
insbesondere wobei die Zusammensetzung mindestens einen Gelbildner aufweist, insbesondere wobei der Gelbildner ausgewählt ist aus der Gruppe von Bentoniten, Kieselsäuren, Polyacrylsäuren, Carbomeren, Polyvinylpyrrolidonen, Cellulose und Cellulosederivaten, Xanthanen sowie deren Mischungen, vorzugsweise Cellulose und Cellulosederivaten; und/oder
insbesondere wobei die Zusammensetzung den Gelbildner in einer relativen Menge im Bereich von 0,01 bis 20 Gew.-%, insbesondere im Bereich 0,1 bis 10 Gew.-%, vorzugsweise im Bereich 0,5 bis 5 Gew.-%, bevorzugt im Bereich von 1 bis 3 Gew.-%, bezogen auf die Zusammensetzung, enthält.

12. Zusammensetzung nach einem der Ansprüche 1 bis 8,
wobei die Zusammensetzung als flüssige Darreichungsform vorliegt und/oder wobei die Zusammensetzung als Fluid, Mundwasser, Mundspülung, Saft, Gurgellösung oder dergleichen vorliegt;
insbesondere wobei die Zusammensetzung wässrig, alkoholisch oder wässrigalkoholisch basiert ist; und/oder
insbesondere wobei die Zusammensetzung Wasser enthält, insbesondere in einer relativen Menge von mindestens 10 Gew.-%, insbesondere mindestens 20 Gew.-%, vorzugsweise mindestens 30 Gew.-%, bezogen auf die Zusammensetzung, und/oder insbesondere in einer relativen Menge im Bereich von 10 bis 98 Gew.-%, insbesondere im Bereich 20 bis 95 Gew.-%, vorzugsweise 30 bis 90 Gew.-%, bezogen auf die Zusammensetzung; und/oder
insbesondere wobei die Zusammensetzung mindestens eine Säure und/oder Base, insbesondere zur Ausbildung eines Puffersystems, enthält und/oder wobei die Zusammensetzung mindestens ein Puffersystem enthält und/oder wobei die Zusammensetzung vorzugsweise mindestens eine Base aufweist, insbesondere wobei die Base ausgewählt ist aus der Gruppe von Aminen, Carboxylaten, Alkali- und/oder Erdalkalimetallhydroxiden sowie deren Mischungen und Kombinationen, insbesondere Alkali- und Erdalkalimetallhydroxiden, bevorzugt Natriumhydroxid; und/oder
insbesondere wobei die Zusammensetzung als Spray, Aerosol oder dergleichen vorliegt.

13. Zusammensetzung, insbesondere pharmazeutische Zusammensetzung, insbesondere nach einem der vorangehenden Ansprüche, vorzugsweise in Form einer festen oder flüssigen, bevorzugt festen Dosierung, insbesondere zur topischen Behandlung von Heiserkeit und/oder entzündlichen Erkrankungen des Mund- und Rachenraums,
wobei die Zusammensetzung in Kombination und jeweils in wirksamen, insbesondere pharmazeutisch wirksamen Mengen
(a) mindestens ein antiviral wirksames Polysaccharid, ausgewählt aus Carrageenen und/oder deren und/oder deren physiologisch verträglichen Salzen, Galactansulfaten und/oder deren physiologisch verträglichen Salzen und Polyuroniden und/oder deren physiologisch verträglichen Salzen, in einer relativen Menge im Bereich von 0,1 bis 40 Gew.-%, insbesondere im Bereich 0,5 bis 30 Gew.-%, vorzugsweise im Bereich von 1 bis 25 Gew.-%, bevorzugt im Bereich von 2 bis 20 Gew.-%, besonders bevorzugt im Bereich von 3 bis 18 Gew.-%, bezogen auf die Zusammensetzung;
(b) Polyhexanid als Oberflächendesinfektionsmittel in einer relativen Menge im Bereich von 0,001 bis 5 Gew.-%, insbesondere im Bereich von 0,002 bis 2,5 Gew.-%, vorzugsweise im Bereich von 0,005 bis 2 Gew.-%, bevorzugt im Bereich von 0,01 bis 1 Gew.-%, besonders bevorzugt im Bereich von 0,05 bis 0,5 Gew.-%, bezogen auf die Zusammensetzung; sowie
(c) Hyaluronsäure oder deren Salze in einer relativen Menge im Bereich von 0,01 bis 10 Gew.-%, insbesondere im Bereich von 0,05 bis 5 Gew.-%, vorzugsweise im Bereich von 0,1 bis 2 Gew.-%, bezogen auf die Zusammensetzung;
aufweist.

14. Zusammensetzung nach einem der vorangehenden Ansprüche
zur Verwendung im Bereich der Pharmazie, Medizin oder Lebensmitteltechnik und/oder
zur Verwendung in der Humanmedizin oder Veterinärmedizin und/oder
zur Verwendung bei der prophylaktischen und/oder therapeutischen Behandlung von Heiserkeit und/oder entzündlichen Erkrankungen des Mund- und Rachenraums, insbesondere zur Verwendung bei der topischen Behandlung von Heiserkeit.

## Claims

1. Composition, in particular a pharmaceutical composition, preferably in the form of a solid or liquid, in particular a solid dosage, particularly for topical treatment of hoarseness or sore throat and/or inflammatory diseases of the mouth and throat,
wherein the composition - in combination and in each case in effective, particularly pharmaceutically effective, amounts - contains:
(a) at least an antiviral, and/or antivirally effective, polysaccharide selected from carrageenans and/or their physiologically acceptable salts, galactan sulfates and/or their physiologically acceptable salts and polyuronides and/or their physiologically acceptable salts ("component (a)");
(b) polyhexanide as a surface disinfectant, ("component (b)"); as well as
(c) hyaluronic acid or salts thereof ("component (c)").

2. Composition according to claim 1,
wherein component (a) has a weight average molecular weight in the range of 5,000 to 10,000,000 Da, particularly in the range of 15,000 to 7,500,000 Da, preferably in the range of 20,000 to 5,000,000 Da, more preferably in the range of 30,000 to 3,000,000 Da, in particular, determined by gel permeation chromatography (GPC), preferably according to DIN 55672; and/or
wherein component (a) is a biopolymer and/or a polymer of biological origin; and/or
wherein component (a) is selected from homopolysaccharides and/or heteropolysaccharides, preferably heteropolysaccharides.

3. Composition according to any one of the preceding claims,
wherein component (a), in particular the polysaccharide, is preferably provided as a mucus substance, preferably in the form of a drug, in particular in the form of comminuted or pulverized or extracted algae, plant parts or plant constituents; and/or
wherein component (a), in particular the polysaccharide, is preferably provided as a mucus substance, preferably in the form of a an extract, in particular in the form of a dry extract, in particular wherein the extract is obtained from an aqueous, alcoholic or aqueous-alcoholic extract, preferably an aqueous extract; in particular wherein the extract, preferably the dry extract, has a drug/extract ratio (DER), in particular calculated as a weight-related ratio of starting amounts of drug to the extract to be obtained, in the range of 0.1:1 to 50:1, particularly in the area of 0.5:1 to 25:1, preferably in the range of 2:1 to 20:1, more preferably in the range of 5:1 to 10:1.

4. Composition according to any one of the preceding claims,
wherein component (a), in particular, the polysaccharide, preferably the mucus substance, is obtainable from Icelandic moss *(Lichen islandicus*), marshmallow *(Althaea officinalis L.),* plantain *(Plantago lanceolata L.),* mallow *(Malva sylvestris L.* and *M. neglecta WALLR),* fenugreek (*Trigonella foenum-graecum L.),* salep, quince *(Cydonia oblonga MILL.*) and algae, preferably red algae, especially red algae species of the genera *chondrus, euchema* and/or *gigartina,* as well as combinations thereof; and/or
wherein component (a) is selected from the alpha, iota, gamma, kappa and/or lambda-carrageenan and/or derivatives thereof and/or their physiologically acceptable salts, preferably iota-carrageenan and/or derivatives thereof and/or their physiologically compatible salts, and/or wherein component (a), especially the polysaccharide, preferably the mucus substance, is a hetero polymer based on iota alpha, gamma, kappa and/or lambda-carrageenan and/or derivatives thereof and/or their physiologically acceptable salts, in particular on the basis of iota and kappa carrageenan and/or derivatives thereof and/or their physiologically acceptable salts; and/or
wherein component (a), especially the polysaccharide, preferably the mucus substance, is obtainable by extraction from algae, preferably red algae, more preferably red algal species of the genera *chondrus, euchema* and/or *gigartina.*

5. Composition according to any one of the preceding claims,
wherein the composition contains component (a) in a relative amount in the range of 0.1 to 40 wt.-%, in particular in the range of 0.5 to 30 wt.-%, preferably in the range of 1 to 25 wt.-% , more preferably in the range of 2 to 20 wt.-%, particularly preferably in the range of 3 to 18 wt.-%, based on the composition; and/or
wherein the composition contains component (a) in an absolute amount in the range of 0.001 to 5 g, particularly in the range of 0.005 to 4 g, preferably in the range of 0.01 to 3 g, more preferably in the range of 0.05 to 2 g, in particular in relation to an application unit, and/or in particular to an application size; and/or
wherein the composition contains component (b) in an amount ranging from 0.001 to 5 wt.-%, in particular in the range of 0.01 to 2.5 wt.-%, preferably in the range of 0.05 to 2 wt.-%, more preferably in the range of 0.01 to 1 wt.-%, particularly preferably in the range of 0.05 to 0.5 wt.-%, based on the composition.

6. Composition according to any one of the preceding claims,
wherein component (c) is the sodium salt of hyaluronic acid; and/or
wherein the composition contains component (c) in an amount ranging from 0.01 to 10 wt.-%, in particular in the range of 0.05 to 5 wt.-%, preferably in the range of 0.1 to 1 wt.-%, based on the composition; and/or
wherein the composition contains component (d) in a relative amount in the range of 0.01 to 5 wt.-%, in particular in the range of 0.05 to 2.5 wt.-%, preferably in the range of 0.01 to 1.5 wt.-%, more preferably in the range of 0.1 to 1 wt.-%, based on the pharmaceutical composition; and/or
wherein the composition has at least a local anesthetic as an additional component (d); and/or
in particular wherein component (d) is selected from the group of polidocanol, benzocaine, lidocain and/or ambroxol, in particular wherein component (d) is polidocanol; and/or
in particular wherein the composition contains component (d) in a relative amount in the range of 0.01 to 5 wt.-%, in particular in the range of 0.05 to 2.5 wt.-%, preferably in the range of 0.01 to 1.5 wt .-%, more preferably in the range of 0.1 to 1 wt.-%, based on the pharmaceutical composition.

7. Composition according to any one of the preceding claims,
wherein the composition contains component (d) in an absolute amount in the range of 0.01 to 500 mg, in particular in the range of 0.05 to 250 mg, preferably in the range of 0.1 to 150 mg, more preferably in the range of 0.5 to 50 mg, particularly preferably in the range of 1 to 20 mg, based on an application unit; and/or
wherein the composition contains component (a) and component (b) in a weight ratio of ([a]: [b]) in the range from 1:1 to 1,000:1, in particular in the range from 5:1 to 500:1, preferably in the range from 15:1 to 100:1, more preferably in the range from 20:1 to 70:1, and/or
wherein the composition comprises component (a) and component (c) in a weight ratio of ([a]: [c]) in the range from 2:1 to 500:1, in particular in the range from 3:1 to 100:1, preferably in the range from 5:1 to 50:1, more preferably in the range from 9:1 to 30:1, and/or
wherein the composition contains component (b) and component (c) in a weight ratio of ([b]: [c]) in the range of 1:1 to 1:10, in particular in the range of 1:2 to 1:4.

8. Composition according to any one of the preceding claims,
wherein the composition contains at least one further active substance and/or ingredient, in particular selected from the group of mucosal protective agents, antiseptics, vitamins, trace elements, minerals, micronutrients and mixtures thereof; and/or
wherein the composition further contains at least one additive, in particular selected from the group consisting of processing aids, colorants, buffers, fragrances, perfumes, extenders, binders, wetting agents and/or preservatives, pH adjusters, pH buffering agents, thickeners, flavors, aromas, sweeteners and sweetening agents, acidifiers, stabilizers and/or antiseptics and mixtures thereof; in particular wherein the composition has glycerin and/or its physiologically acceptable derivatives or ester as an additive, in particular in an amount in the range of 0.01 to 5 wt.-%, in particular in the range of 0.05 to 4 wt.-%, preferably in the range of 0.1 to 2.5 wt.-%, more preferably in the range of 0.2 to 1 wt.-%, based on the pharmaceutical composition.

9. Composition according to any one of the preceding claims, wherein the composition is present as a solid dosage form and/or wherein the composition is present as a tablet, dragee, pill, hard caramel or the like, in particular as a lozenge, in particular wherein the lozenge has a total weight in the range of 0.5 to 5 g, in particular in the range of 0.8 to 4 g, preferably in the range of 1 to 3 g;
in particular wherein the composition contains the active substances and/or ingredients in a solid matrix and/or mass, in particular in a matrix and/or composition based on sugars and/or sugar substitutes, in particular where the sugars are selected from the group of sucrose, glucose, in particular dextrose, and fructose and/or in particular wherein the sugar substitutes are selected from sugar alcohols, preferably from the group of mannitol, xylitol, sorbitol, isomalt, maltitol syrup, lactitol, leucrose, fructooligosaccharides, glucans, polyglucose, particularly preferably isomalt; and/or
in particular wherein the composition contains sugar and/or sugar substitutes, in a relative amount in the range of 30 to 99.9 wt.-%, in particular in the range of 40 to 99 wt.-%, preferably in the range of 50 to 98 wt %, based on the composition; and/or
in particular wherein the solid dosage form, especially a lozenge, is so formed that the solid dosage form, especially a lozenge, releases a therapeutically effective amount of the active ingredients and/or substances on sucking when the solid dosage form is administered in the mouth and throat of a patient and is sucked.

10. Composition according to any one of the claims 1 to 8, wherein the composition is present as a viscous and/or paste-like administration form and/or wherein the composition is present as an ointment, cream, paste, gel or the like;
in particular wherein the composition is aqueous, alcoholic or aqueous-alcoholic or organic based; and/or
in particular wherein the composition contains water, in particular in a relative amount of least 10 wt.-%, in particular at least 20 wt.-%, preferably at least 30 wt.-%, based on the composition, and/or in particular in a relative amount in the range of 10 to 90 wt.-%, preferably in the range of 20 to 85 wt .-%, more preferably in the range of 30 to 80 wt.-%, based on the composition; and/or
in particular wherein the composition contains at least one excipient, particularly wherein the excipient is selected from the group of solvents, solubilizers, emulsifiers, and the like, and/or wherein the carrier is selected from the group of ethanol, isopropanol, ethyl carbonate, ethyl acetate, benzyl alcohol, benzyl benzoate , propylene glycol, 1,3-butyl glycol, as well as combinations thereof.

11. Composition according to claim 10,
wherein the composition contains at least one excipient, particularly wherein the excipient is an oil and/or fat and/or in particular wherein the carrier is selected from wool grease, cottonseed oil, vaseline, glycerol fatty acid esters, polyethylene glycols, and combinations thereof;
in particular wherein at a temperature of 25°C the composition has a Brookfield viscosity in the range of 500 to 100,000 mPas, particularly in the range of 750 to 50,000 mPas, preferably in the range of 1,000 to 10,000 mPas, particularly preferably in the range of 1,500 to 8,000 mPas, very particularly preferably in the range 2,000 to 6,000 mPas; and/or
in particular wherein the composition contains at least one gelling agent, in particular wherein the gelling agent is selected from the group of bentonites, silicic acids, polyacrylic acids, carbomers, polyvinyl pyrrolidones, cellulose and cellulose derivatives, xanthans and mixtures thereof, preferably cellulose and cellulose derivatives; and/or
in particular wherein the composition preferably contains the gelling agent in a relative amount in the range of 0.01 to 20 wt.-%, in particular in the range 0.1 to 10 wt.-%, preferably in the range 0.5 to 5 wt.-%, more preferably in the range of 1 to 3 wt .-%, based on the composition.

12. Composition according to any one of the claims 1 to 8,
wherein the composition is present as a liquid dosage form and/or the composition is present as a fluid, mouth wash, mouth rinse, juice, gargling solution or the like;
in particular wherein the composition is aqueous, alcoholic or aqueous-alcoholic based; and/or
in particular wherein the composition contains water, in particular in a relative amount of least 10 wt.-%, in particular at least 20 wt.-%, preferably at least 30 wt.-%, based on the composition, and/or in particular in a relative amount in the range of 10 to 98 wt.-%, in particular in the range 20 to 95 wt.-%, preferably in the range of 30 to 90 wt.-%, based on the composition; and/or
in particular wherein the composition contains at least one acid and/or base, in particular for forming a buffer system, and/or wherein the composition contains at least a buffer system, and/or wherein the composition preferably comprises at least a base, in particular wherein the base is selected from the group of amines, carboxylates, alkali and/or alkaline earth metal hydroxides as well as mixtures and combinations thereof, in particular alkali and alkaline earth metal hydroxides, preferably sodium hydroxide; and/or
in particular wherein the composition is present as a spray, aerosol or the like.

13. Composition, in particular a pharmaceutical composition, in particular according to one of the preceding claims, preferably in the form of a solid or liquid, preferably a solid dosage, in particular for the topical treatment of hoarseness and/or inflammatory diseases of the mouth and throat,
wherein the composition - in combination and in each case in effective, particularly pharmaceutically effective, amounts - contains:
(a) at least an antiviral, and/or antivirally effective, polysaccharide selected from carrageenans and/or their physiologically acceptable salts, galactan sulfates and/or their physiologically acceptable salts and polyuronides and/or their physiologically acceptable salts, in a relative amount in the range of 0.1 to 40 wt.-%, in particular in the range of 0.5 to 30 wt.-%, preferably in the range of 1 to 25 wt.-%, more preferably in the range of 2 to 20 wt.-%, particularly preferably in the range of 3 to 18 wt.-%, based on the composition;
(b) polyhexanide as a surface disinfectant in a relative amount in the range of 0.001 to 5 wt.-%, in particular in the range of 0.002 to 2.5 wt.-%, preferably in the range of 0.005 to 2 wt.-%, more preferably in the range of 0.01 to 1 wt.-%, particularly preferably in the range of 0.05 to 0.5 wt.-%, based on the composition: and
(c) hyaluronic acid or salts thereof in a relative amount in the range of 0.01 to 10 wt.-%, in particular in the range of 0.05 to 5 wt.-%, preferably in the range of 0.1 to 2 wt.-%, based on the composition.

14. Composition according to any one of the preceding claims
for use in the field of pharmacy, medicine or food technology, and/or
for use in human medicine or veterinary medicine, and/or
for use in the prophylactic and/or therapeutic treatment of hoarseness and/or inflammatory diseases of the mouth and throat, especially for use in the topical treatment of huskiness.

## Revendications

1. Composition, en particulier composition pharmaceutique, de préférence sous forme d'une posologie solide ou liquide, en particulier solide, en particulier pour le traitement topique de l'enrouement ou des douleurs du cou et/ou des maladies inflammatoires de la cavité buccale et du pharynx,
la composition comprenant - en association, et chacun en des quantités efficaces, en particulier pharmaceutiquement efficaces -
(a) au moins un polysaccharide virostatique et/ou à activité antivirale, choisi parmi les carragènes et/ou leurs sels physiologiquement compatibles, les sulfates de galactane et/ou leurs sels physiologiquement compatibles, et les polyuronides et/ou leurs sels physiologiquement compatibles ("composant (a)") ;
(b) un polyhexanide en tant que désinfectant superficiel ("composant (b)") ; ainsi que
(c) de l'acide hyaluronique ou ses sels ("composant (c)").

2. Composition selon la revendication 1,
dans laquelle le composant (a) présente une masse moléculaire moyenne en masse comprise dans la plage de 5 000 à 10 000 000 Da, en particulier dans la plage de 15 000 à 7 500 000 Da, de préférence dans la plage de 20 000 à 5 000 000 Da, préférentiellement dans la plage de 30 000 à 3 000 000 Da, déterminée en particulier par chromatographie par perméation de gel (GPC), de préférence selon DIN 55672 ; et/ou
le composant (a) étant un biopolymère et/ou un polymère d'origine biogène ; et/ou
le composant (a) étant choisi parmi les homopolysaccharides et/ou les hétéropolysaccharides, de préférence les hétéropolysaccharides.

3. Composition selon l'une des revendications précédentes, dans laquelle le composant (a), en particulier le polysaccharide, est mis à disposition de préférence sous forme d'une substance mucilagineuse, de préférence sous forme d'une drogue, en particulier sous forme d'algues, de parties de plantes ou de constituants de plantes, broyés ou pulvérisés ou extraits,
le composant (a), en particulier le polysaccharide, étant mis à disposition en particulier sous forme d'une substance mucilagineuse, de préférence sous forme d'un extrait, en particulier d'un extrait sec, en particulier l'extrait sec pouvant être obtenu à partir d'un extrait aqueux, alcoolique ou hydro-alcoolique, de préférence d'un extrait aqueux, en particulier l'extrait, de préférence l'extrait sec, présentant un rapport drogue/extrait (DEV), en particulier calculé par le rapport, en poids, de la quantité initiale utilisée de drogue à celle de l'extrait obtenu, compris dans la plage de 0,1:1 à 50:1, en particulier dans la plage de 0,5:1 à 25:1, de préférence dans la plage de 2:1 à 20:1, d'une manière particulièrement préférée dans la plage de 5:1 à 10:1.

4. Composition selon l'une des revendications précédentes,
dans laquelle le composant (a), en particulier le polysaccharide, de préférence la substance mucilagineuse, peut être obtenu à partir de mousse d'Islande (*Lichen islandicus*), de guimauve officinale (*Althaea officinalis L*.), de plantain lancéolé (*Plantago lanceolata L*.), de mauve (*Malva sylvestris L.* et *M. neglecta WALLR*.), de trigonelle fénugrec (*Trigonella foenum-graecum L*.), de salep, de coing (*Cydonia oblonga MILL*.) et d'algues, de préférence d'algues rouges, en particulier des espèces d'algues rouges des genres *Chondrus, Euchema* et/ou *Gigartina,* ainsi que de leurs combinaisons ; et/ou
le composant (a) étant choisi parmi les carragènes alpha, iota, gamma, kappa et/ou lambda et/ou leurs dérivés et/ou leurs sels physiologiquement compatibles, de préférence le carragène iota et/ou ses dérivés et/ou ses sels physiologiquement compatibles, et/ou le composant (a), en particulier le polysaccharide, de préférence la substance mucilagineuse, étant un hétéropolymère à base de carragènes alpha, iota, gamma, kappa et/ou lambda et/ou de leurs dérivés et/ou de leurs sels physiologiquement compatibles, en particulier à base de carragènes iota et kappa et/ou de leurs dérivés et/ou de leurs sels physiologiquement compatibles ; et/ou
le composant (a), en particulier le polysaccharide, de préférence la substance mucilagineuse, pouvant être obtenu par extraction d'algues, de préférence d'algues rouges, d'une manière particulièrement préférée d'espèces d'algues rouges des genres *Chondrus, Euchema* et/ou *Gigartina.*

5. Composition selon l'une des revendications précédentes,
dans laquelle la composition contient le composant (a) en une quantité relative comprise dans la plage de 0,1 à 40 % en poids, en particulier dans la plage de 0,5 à 30 % en poids, de préférence dans la plage de 1 à 25 % en poids, de préférence dans la plage de 2 à 20 % en poids, d'une manière particulièrement préférée dans la plage de 3 à 18 % en poids, par rapport à la composition ; et/ou
la composition contenant le composant (a) en une quantité absolue comprise dans la plage de 0,001 à 5 g, en particulier dans la plage de 0,005 à 4 g, de préférence dans la plage de 0,01 à 3 g, de préférence dans la plage de 0,05 à 2 g, en particulier par rapport à une unité d'administration et/ou en particulier à une quantité d'administration ; et/ou
la composition contenant le composant (b) en une quantité comprise dans la plage de 0,001 à 5 % en poids, en particulier dans la plage de 0,01 à 2,5 % en poids, de préférence dans la plage de 0,05 à 2 % en poids, préférentiellement dans la plage de 0,01 à 1 % en poids, d'une manière particulièrement préférée dans la plage de 0,05 à 0,5 % en poids, par rapport à la composition.

6. Composition selon l'une des revendications précédentes,
dans laquelle le composant (c) est le sel de sodium de l'acide hyaluronique ; et/ou
la composition contenant le composant (c) en une quantité comprise dans la plage de 0,01 à 10 % en poids, en particulier dans la plage de 0,05 à 5 % en poids, de préférence dans la plage de 0,1 à 1 % en poids, par rapport à la composition ; et/ou
la composition contenant le composant (d) en une quantité relative comprise dans la plage de 0,01 à 5 % en poids, en particulier dans la plage de 0,05 à 2,5 % en poids, de préférence dans la plage de 0,01 à 1,5 % en poids, préférentiellement dans la plage de 0,1 à 1 % en poids, par rapport à la composition pharmaceutique et/ou
la composition contenant en tant que composant supplémentaire (d) au moins un anesthésiant local et/ou
en particulier le composant (d) étant choisi parmi le polidocanol, la benzocaïne, la lidocaïne et/ou l'ambroxol, le composant (d) étant en particulier le polidocanol ; et/ou
en particulier la composition contenant le composant (d) en une quantité relative comprise dans la plage de 0,01 à 5 % en poids, en particulier dans la plage de 0,05 à 2,5 % en poids, de préférence dans la plage de 0,01 à 1,5 % en poids, préférentiellement dans la plage de 0,1 à 1 % en poids, par rapport à la composition pharmaceutique.

7. Composition selon l'une des revendications précédentes,
la composition contenant le composant (d) en une quantité absolue comprise dans la plage de 0,01 à 500 mg, en particulier dans la plage de 0,05 à 250 mg, de préférence dans la plage de 0,1 à 150 mg, préférentiellement dans la plage de 0,5 à 50 mg, d'une manière particulièrement préférée dans la plage de 1 à 20 mg, par rapport à une unité d'administration ; et/ou
la composition comprenant le composant (a) et le composant (b) selon un rapport en poids ([a]:[b]) compris dans la plage de 1:1 à 1 000:1, en particulier dans la plage de 5:1 à 500:1, de préférence dans la plage de 15:1 à 100:1, préférentiellement dans la plage de 20:1 à 70:1, et/ou
la composition comprenant le composant (a) et le composant (c) selon un rapport en poids ([a]:[c]) compris dans la plage de 2:1 à 500:1, en particulier dans la plage de 3:1 à 100:1, de préférence dans la plage de 5:1 à 50:1, préférentiellement dans la plage de 9:1 à 30:1, et/ou
la composition comprenant le composant (b) et le composant (c) selon un rapport en poids ([b]:[c]) compris dans la plage de 1:1 à 1:10, en particulier dans la plage de 1:2 à 1:4.

8. Composition selon l'une des revendications précédentes,
la composition contenant au moins un autre principe actif et/ou constituant, en particulier choisi dans le groupe consistant en les agents protecteurs des muqueuses, les antiseptiques, les vitamines, les oligoéléments, les minéraux, les micronutriments, ainsi que les mélanges de ceux-ci ; et/ou la composition contenant par ailleurs au moins un additif en particulier choisi dans le groupe consistant en les auxiliaires de mise en oeuvre, les substances colorantes, les tampons, les aromatisants, les parfums, les diluants, les liants, les mouillants et/ou les conservateurs, les agents d'ajustement du pH, les substances tampons de pH, les épaississants, les arômes, les agents de sapidité, les édulcorants et substances sucrantes, les acidifiants, les stabilisants et/ou les antiseptiques, ainsi que les mélanges de ceux-ci ; en particulier la composition comprenant en tant qu'additifs du glycérol et/ou des dérivés ou esters physiologiquement compatibles de ce dernier, en particulier en une quantité comprise dans la plage de 0,01 à 5 % en poids, en particulier dans la plage de 0,05 à 4 % en poids, de préférence dans la plage de 0,1 à 2,5 % en poids, préférentiellement dans la plage de 0,2 à 1 % en poids, par rapport à la préparation pharmaceutique.

9. Composition selon l'une des revendications précédentes, la composition se présentant sous une forme posologique solide, et/ou la composition se présentant sous forme d'un comprimé, d'une dragée, d'une pilule, d'un caramel dur ou analogues, en particulier d'un comprimé à sucer, en particulier dans laquelle le comprimé à sucer présente une masse totale comprise dans la plage de 0,5 à 5 g, en particulier dans la plage de 0,8 à 4 g, de préférence dans la plage de 1 à 3 g ; en particulier la composition comprenant les principes actifs et/ou les constituants dans une matrice et/ou une masse solides, en particulier dans une matrice et/ou une masse à base de sucres et/ou de succédanés de sucre, les sucres étant en particulier choisis dans le groupe consistant en le saccharose, le glucose, en particulier le dextrose, et le fructose, et/ou en particulier les succédanés du sucre étant choisis parmi les alcools de sucre, de préférence dans le groupe consistant en le mannitol, le xylitol, le sorbitol, l'isomaltol, le sirop de maltitol, le lactitol, le leucrose, les fructo-oligosaccharides, les glucanes, le polyglucose, d'une manière particulièrement préférée l'isomaltol ; et/ou en particulier la composition contenant les sucres et/ou les succédanés du sucre en une quantité relative comprise dans la plage de 30 à 99,9 % en poids, en particulier dans la plage de 40 à 99 % en poids, de préférence dans la plage de 50 à 98 % en poids, par rapport à la composition ; et/ou
la forme posologique solide, en particulier le comprimé à sucer, étant formé de telle sorte que la forme posologique solide, en particulier le comprimé à sucer, libère une quantité thérapeutiquement efficace des principes actifs et/ou des constituants lors du suçage, quand la forme posologique solide est administrée et sucée dans la cavité buccale et le pharynx d'un patient.

10. Composition selon l'une des revendication 1 à 8, la composition se présentant sous forme d'une forme posologique visqueuse et/ou pâteuse, la composition se présentant sous forme d'une pommade, d'une crème, d'une pâte, d'un gel ou analogues ;
en particulier la composition étant à base aqueuse, alcoolique, hydro-alcoolique ou organique ; et/ou la composition contenant de l'eau, de préférence en une quantité relative d'au moins 10 % en poids, en particulier d'au moins 20 % en poids, de préférence d'au moins 30 % en poids, par rapport à la composition, et/ou en particulier en une quantité relative comprise dans la plage de 10 à 90 % en poids, en particulier dans la plage de 20 à 85 % en poids, de préférence dans la plage de 30 à 80 % en poids, par rapport à la composition : et/ou
en particulier la composition contenant au moins un support, en particulier le support étant choisi dans le groupe des solvants, des tiers-solvants, des émulsifiants et analogues, et/ou le support étant choisi dans le groupe consistant en l'éthanol, l'isopropanol, le carbonate d'éthyle, l'acétate d'éthyle, l'alcool benzylique, le benzoate de benzyle, le propylèneglycol, le 1,3-butylglycol, ainsi que les combinaisons de ceux-ci.

11. Composition selon la revendication 10,
la composition contenant au moins un support, en particulier le support étant une huile et/ou une graisse, et/ou en particulier le support étant choisi parmi le suint, l'huile de graines de coton, la vaseline, les esters d'acides gras du glycérol, les polyéthylèneglycols, ainsi que les combinaisons de ceux-ci ;
en particulier la composition présentant à une température de 25°C une viscosité Brookfield comprise dans la plage de 500 à 100 000 mPa.s, en particulier dans la plage de 750 à 50 000 mPa.s, préférentiellement dans la plage de 1 000 à 10 000 mPa.s, d'une manière particulièrement préférée dans la plage de 1 500 à 8 000 mPa.s, d'une manière tout particulièrement préférée dans la plage de 2 000 à 6 000 mPa.s ; et/ou
en particulier la composition comprenant au moins un gélifiant, le gélifiant étant en particulier choisi dans le groupe consistant en les bentonites, les silices, les poly(acides acryliques), les Carbomers, les polyvinylpyrrolidones, la cellulose et les dérivés de la cellulose, les xanthanes et leurs mélanges, de préférence la cellulose et les dérivés de la cellulose ; et/ou en particulier la composition contenant le gélifiant en une quantité relative comprise dans la plage de 0,01 à 20 % en poids, en particulier dans la plage de 0,1 à 10 % en poids, de préférence dans la plage de 0,5 à 5 % en poids, préférentiellement dans la plage de 1 à 3 % en poids, par rapport à la composition.

12. Composition selon l'une des revendications 1 à 8,
la composition se présentant sous une forme posologique liquide, et/ou la composition se présentant sous forme d'un fluide, d'un collutoire, d'un bain de bouche, d'un jus, d'une solution pour gargarisme ou analogues ;
en particulier la composition étant à base aqueuse, alcoolique ou hydro-alcoolique ; et/ou
en particulier la composition contenant de l'eau, en particulier en une quantité relative d'au moins 10 % en poids, en particulier d'au moins 20 % en poids, de préférence d'au moins 30 % en poids, par rapport à la composition, et/ou en particulier en une quantité relative comprise dans la plage de 10 à 98 % en poids, en particulier dans la plage de 20 à 95 % en poids, de préférence de 30 à 90 % en poids, par rapport à la composition ; et/ou
en particulier la composition contenant au moins un acide et/ou une base, en particulier pour former un système tampon, et/ou la composition contenant au moins un système tampon, et/ou la composition comprenant de préférence au moins une base, en particulier la base étant choisie dans le groupe consistant en les amines, les carboxylates, les hydroxydes de métaux alcalins et/ou alcalino-terreux, ainsi que les mélanges et combinaisons de ceux-ci, en particulier les hydroxydes de métaux alcalins et alcalino-terreux, de préférence l'hydroxyde de sodium ; et/ou
en particulier la composition se présentant sous forme d'un spray, d'un aérosol ou analogues.

13. Composition, en particulier composition pharmaceutique, en particulier selon l'une des revendications précédentes, de préférence sous forme d'une posologie solide ou liquide, de préférence solide, en particulier pour le traitement topique de l'enrouement et/ou des maladies inflammatoires de la cavité buccale et du pharynx,
la composition comprenant, en association et chacun en une quantité efficace, en particulier pharmaceutiquement efficace
(a) au moins un polysaccharide à effet antiviral, choisi parmi les carragènes et/ou leurs sels et/ou leurs sels physiologiquement compatibles, les sulfates de galactane, et/ou leurs sels physiologiquement compatibles, et les polyuronides et/ou leurs sels physiologiquement compatibles, en une quantité relative comprise dans la plage de 0,1 à 40 % en poids, en particulier dans la plage de 0,5 à 30 % en poids, de préférence dans la plage de 1 à 25 % en poids, préférentiellement dans la plage de 2 à 20 % en poids, d'une manière particulièrement préférée dans la plage de 3 à 18 % en poids, par rapport à la composition ;
(b) du polyhexanide en tant que désinfectant superficiel, en une quantité relative comprise dans la plage de 0,001 à 5 % en poids, en particulier dans la plage de 0,002 à 2,5 % en poids, de préférence dans la plage de 0,005 à 2 % en poids, préférentiellement dans la plage de 0,01 à 1 % en poids, d'une manière particulièrement préférée dans la plage de 0,05 à 0,5 % en poids, par rapport à la composition ; ainsi que
(c) de l'acide hyaluronique ou ses sels, en une quantité relative comprise dans la plage de 0,01 à 10 % en poids, en particulier dans la plage de 0,05 à 5 % en poids, de préférence dans la plage de 0,1 à 2 % en poids, par rapport à la composition.

14. Composition selon l'une des revendications précédentes pour une utilisation dans le domaine de la pharmacie, de la médecine ou de l'industrie alimentaire, et/ou
pour une utilisation en médecine humaine et/ou en médecine vétérinaire, et/ou
pour une utilisation lors du traitement prophylactique et/ou thérapeutique de l'enrouement et/ou des maladies inflammatoires de la cavité buccale et du pharynx, en particulier pour une utilisation lors du traitement topique de l'enrouement.
